# EUROPEAN PATENT SPECIFICATION

(11) **EP 3 445 348 B1**
(45) Date of publication and mention of the grant of the patent: **07.06.2023**
(21) Application number: 17721859.1
(22) Date of filing: 18.04.2017
(51) Int. Cl.: A61K 31/12, G01N 33/50, G01N 21/64, A61P 25/28, A61P 3/00

(54) **FLUORESCENT COMPOUNDS, PRODUCTION METHODS AND USES THEREOF**
FLUORESZENZVERBINDUNGEN, IHRE HERSTELLUNG UND VERWENDUNG
COMPOSÉS FLUORESCENTS, LEUR PRÉPARATION ET UTILISATION

(30) Priority: 18.04.2016 PT 2016109330
(43) Date of publication of application: 27.02.2019
(73) Proprietor: Universidade de Aveiro, 3810-193 Aveiro (PT)
(72) Inventor: SOARES DA SILVA, Artur Manuel, 3800-157 Aveiro (PT); ANDRÉ GUIEU, Samuel Jacques, 3800-153 Aveiro (PT); MATIAS CELESTINO GOMES DA ROCHA, João Carlos, 3830-005 Ílhavo (PT); MOREIRA PINTO VIEIRA, Sandra Isabel, 3810-055 Aveiro (PT); DA CRUZ E SILVA, Odete, 2135-297 Samora Correia (PT); DOS SANTOS DIAS, Roberto Alexandre, 2420-084 Caranguejeira (PT); FRADE RUIVO, Raquel, 3800-153 Aveiro (PT)
(74) Representative: Patentree
(86) International application number: PCT/IB2017/052209
(87) International publication number: WO 2017/182945

(56) References cited:
- LI ZIJING ET AL: "Novel F-18-labeled dibenzylideneacetone derivatives as potential positron emission tomography probes for in vivo imaging of beta-amyloid plaque", JOURNAL OF LABELLED COMPOUNDS AND RADIOPHARMACEUTICALS, vol. 56, no. Suppl. 1, May 2013 (2013-05), page S285, XP002771270, & 20TH INTERNATIONAL SYMPOSIUM ON RADIOPHARMACEUTICAL SCIENCES; JEJU, SOUTH KOREA; MAY 12 -17, 2013
- YANG YANPING ET AL: "99mTc-labeled dibenzylideneacetone derivatives as potential SPECT probes forin vivoimaging of[beta]-amyloid pla", EUROPEAN JOURNAL OF MEDICINAL CHEMISTRY, EDITIONS SCIENTIFIQUE ELSEVIER, PARIS, FR, vol. 64, 6 April 2013 (2013-04-06), pages 90-98, XP028566321, ISSN: 0223-5234, DOI: 10.1016/J.EJMECH.2013.03.057
- CUI MENGCHAO ET AL: "Synthesis and Structure-Affinity Relationships of Novel Dibenzylideneacetone Derivatives as Probes for beta-Amyloid Plaques", JOURNAL OF MEDICINAL CHEMISTRY, vol. 54, no. 7, April 2011 (2011-04), pages 2225-2240, XP002771271, ISSN: 0022-2623
- RAHUL BALASAHEB AHER ET AL: "Dibenzylideneacetone analogues as novelinhibitors", BIOORGANIC & MEDICINAL CHEMISTRY LETTERS, PERGAMON, AMSTERDAM, NL, vol. 21, no. 10, 10 March 2011 (2011-03-10), pages 3034-3036, XP028208774, ISSN: 0960-894X, DOI: 10.1016/J.BMCL.2011.03.037 [retrieved on 2011-03-26]
- KAATZ P & SHELTON D P: "Polarized hyper-Rayleigh light scattering measurements of nonlinear optical chromophores", JOURNAL OF CHEMICAL PHYSICS, AMERICAN INSTITUTE OF PHYSICS, US, vol. 105, no. 10, 1 January 1996 (1996-01-01), pages 3918-3929, XP009194702, ISSN: 0021-9606, DOI: 10.1063/1.472264
- RUANWAS P ET AL: "Fluorescence properties of dienone derivatives and solvent effects on their fluorescence absorption and emission", OPTICS AND SPECTROSCOPY, M A I K NAUKA - INTERPERIODICA, RU, vol. 118, no. 1, 23 January 2015 (2015-01-23), pages 67-72, XP035432999, ISSN: 0030-400X, DOI: 10.1134/S0030400X15010208 [retrieved on 2015-01-23]
- NUNEZ J. ET AL.: "Photophysical study of the Dibenzylideneacetones and 3-Benzylidenethiochroman-4-ones", JOURNAL OF COMPOUTATIONAL METHODS IN SCIENCES AND INGINEERING, vol. 12, 2012, pages 293-298, XP009194711,
- FATHIMA A ANIS ET AL: "Absorption, fluorescence studies and ab initio calculations on binary mixture of p-dimethylaminobenzaldehyde.", JOURNAL OF FLUORESCENCE MAR 2008, vol. 18, no. 2, March 2008 (2008-03), pages 383-391, XP002771272, ISSN: 1053-0509
- TAKATSU MASAHISA ET AL: "Vapor-phase Staining of Cyanoacrylate-Fumed Latent Fingerprints Using p-Dimethylaminobenzaldehyde", JOURNAL OF FORENSIC SCIENCES, vol. 57, no. 2, March 2012 (2012-03), pages 515-520, XP002771858, ISSN: 0022-1198

## Description

### Technical domain

The present disclosure relates to a compound for use in the diagnostics of chronic stress, a lipid-related metabolic disease, lipid-related metabolic disorder, a demyelinating disease or demyelinating disorder.

### Technical background

Fluorescence microscopy and fluorescence imaging are expanding areas of research, in particular medical science research and biological science research.

The fluorescent probes are standard in many applications, and their use is continually increasing due to their versatility, sensitivity and quantitative capabilities. These probes are employed, for example, to light up cells in cultures and *in vivo,* detect location and activation of proteins and other molecules, identify molecular complexes formation, molecular conformational changes and monitor biological processes in vivo. The ability to detect biological molecules in response to environmental changes may also be based on fluorescent methods. Therefore, the demand for more sensitive, more specific and more versatile fluorescent reagents is increasing daily.

These facts are disclosed in order to illustrate the technical problem addressed by the present disclosure.

### Brief description

The present disclosure relates to a family of fluorophores, which permeates cell membranes enabling the labelling of fixed or living cells in cultures and *in vivo,* without the requirement of secondary agents for detection. The present disclosure relates to a compound, in particular, to a fluorescent marker/probe for general cellular and organismal labelling and imaging, specific organelle and molecular staining, and for the comparative analysis of subcellular organelles polarities. The present disclosure also relates to the synthesis of fluorescent reagents combining an emissive centre with halogen atoms. The markers/probes now disclosed may be used to stain and analyse biological samples such as, but not limited to, cells, subcellular organelles, tissues, or entire entities, either *in vivo* or *in vitro.*

The combination of the fluorescent markers/probes now disclosed with a fluorescent microscope allows to visualize fixed and live cells, to perceive the cell contours, to identify subcellular organelles and co-localize other molecules, in particular to use these probes as organelle markers, to analyse the cellular and subcellular morphology and polarity, and to detect regions enriched in more hydrophobic molecules, such as lipids and molecules containing lipid moieties. The compounds now disclosed also allow monitoring the above-mentioned cellular and subcellular structures over time, and to assess potential morphological and even chemical polarity changes over extended periods of time. The fluorescent markers/probes now disclosed have high environmental stability, good quantum yields for fluorescence, large Stokes shifts, and good photo stability.

The advantage of a fluorescent marker/probe with a large Stokes shift is the clear distinction between the excitation and the emission light in a given sample, while a fluorophore with a small Stokes shift is responsible for a greater background signal as a result of a smaller difference between excitation and emission wavelengths. Therefore, the fluorescent marker/probe with a larger Stokes shift eliminates spectral overlap between absorption and emission wavelengths and allows detection of fluorescence while reducing interference. This also eliminates quenching of the fluorescence and gives a stronger signal when used for biological labelling and imaging.

The Stokes shift is the wavelength distance between the excitation and emission wavelengths of a given compound. This aspect is of importance in the detection of the emitted fluorescence in biological applications. The Stokes shift is also a distinct characteristic of each fluorophore. For example, the detection of emitted fluorescence can be difficult to distinguish from the excitation light when using fluorophores with very small Stokes shifts because the excitation and emission wavelengths greatly overlap. Conversely, fluorophores with large Stokes shifts are easy to distinguish because of the large separation between the excitation and emission wavelengths. The Stokes shift is especially critical in multiplex fluorescence applications, because the emission wavelength of one fluorescence marker/probe may overlap, and therefore excite, another fluorescence in the same sample.

The present disclosure relates to compounds having the general formula (1), their synthesis and their use as fluorescent cell and organelle markers, and as molecular tools to analyse their polarity for cellular labelling and imaging. The compounds now disclosed are responsible, in particular, for the visualization, analysis and characterization of biological samples, including fluorescence-related techniques of organismal, cell, subcellular and molecular detection and imaging, and their use as organelle markers. 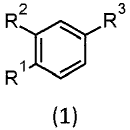 wherein
R¹ is selected from the following list: N(CH₃)₂;
R² is selected from the following list: H or halogen;
R³ is selected from the following list: 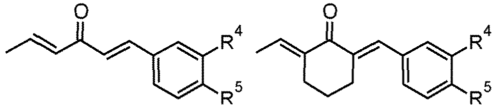
R⁴ is selected from the following list: H or halogen;
R⁵ is selected from the following list: N(CH₃)₂ or OCH₃ and
R¹, R², R³, R⁴, R⁵ are independently selected;
for use in the diagnostics of chronic stress, a lipid-related metabolic disease, lipid-related metabolic disorder, a demyelinating disease or demyelinating disorder;
wherein the demyelinating disease or demyelinating disorder is selected from the following list: multiple sclerosis, acute disseminated encephalomyelitis, Balo's disease, HTLV-I associated myelopathy, neuromyelitis optica, Schilder's disease, transverse myelitis, or cobalamin-deficiencies.

These compounds are stable, have good fluorescence efficiency, in particular good quantum-yields and a large Stokes shift between their excitation and emission maxima. In cells, these compounds selectively accumulate in subcellular organelles such as the Golgi apparatus/Golgi area, nucleus and lipid-enriched regions/organelles such as lipid droplets, and their fluorescence emission alters with the polarity of the solvent used to dissolve the compound. The compounds can be used for selective cellular, subcellular and molecular labelling, imaging, and analysis.

These compounds also present solvatochromism, depending on the solvent polarity. The solvatochromism is defined as the ability of a chemical substance to change its colour; by increasing the solvent polarity, a shift in emission wavelength of the probe occurs, and this can be observed inside the cells.

The dyes currently available on the market present low Stokes shift, few present solvatochromic properties, and none presents solvatochromism toward shorter wavelengths with the increase of hydrophobicity (decreased polarity).

The compounds now disclosed present the following advantages in comparison with the already known prior art fluorescent dyes:
- may be used as a 'add on' dye or a 'ready to use' dye and not a fluorophore-labelled antibody or genome-transforming fluorophore probe as the green fluorescent protein (GFP);
- are easier to manufacture than other fluorescent markers such as boron-dipyrromethene (BODIPY) and 9-diethylamino-5H-benzo[a]phenoxazine-5-one (Nile Red);stain intracellular membranes;
- stain the Golgi area;
- stain the nuclear area;
- stain lipids, lipid-enriched molecules and lipid-enriched regions/organelles;
- have solvatochromism with the polarity of the environment, similar to Nile Red, but in an opposite direction: with the increase of hydrophobicity Nile Red emission shifts to longer wavelengths while the emission of the compounds here disclosed shift to lower emission wavelengths;
- may be used to detect lipids and lipid-enriched molecules, to compare the hydrophobicity of subcellular organelles and to detect alterations in the lipid composition of cellular membranes, alone or in combination with Nile Red, in particular as a complementary probe, which is highly relevant as lipids account for nearly half of the mass of cell membranes;
- highly membrane-permeant, in particular it enters very fast inside cells in culture within 15 minutes - 90 minutes, preferably 30 - 60 minutes;
- not toxic for cells and small organisms, even after long periods (24-48h);
- can stain live cells and organisms - although Nile Red can be used in live cells, usually Nile Red and DHP are used in fixed cells, and in overnight protocols;
- may be used in translationally silent cells, such as the spermatozoa;
- used in the same working concentrations range as Nile red, BODIPY and DHP, in particular 1-10×10⁻⁵ to 1-10×10⁻⁷, preferably 1-10×10⁻⁶ mol.L⁻¹;
- as their emission are low or even virtually absent in the blue region, the compounds are compatible with the most relevant and worldwide used nuclear counterstaining blue-emitting dyes DAPI or Hoechst. In contrast, DHP (cheaper than Nile Red and BODIPY) is not compatible with these blue dyes as it fluoresces in blue;
- DHP working solution is also very difficult to prepare; and DHP is a blue emission fluorophore; therefore more difficult to observe/image;
- lower price: as lipid marker, the price of the disclosed compounds can be at least 100 times lower than DHP or Nile Red and 10 000 xs lower than BODIPY;
- lower price as Golgi marker than any antibody against Golgi proteins, and easier and faster to use.

There are currently available several different types of probes for cellular labelling and imaging. However, these probes present disadvantages, in particular these probes may have low staining efficiency, be cytotoxic when used in live cell imaging, and fail to detect environmental polarity changes. Furthermore, the disadvantages of the previous mentioned compounds also include: being more expensive; their stock solutions can be difficult to prepare; possess smaller Stokes shifts that difficult the separation between excitation and emission lights; none has solvatochromism to the left, in particular from longer to lower wavelengths with the decrease of polarity.

Moreover, the disclosed compounds are highly cell membrane-permeable, selectively accumulate in subcellular regions/organelles, can be used for selective labelling and imaging, and for the characterization of the subcellular relative polarity.

The ability to mark cells and organisms, and to distinguish, identify and characterize subcellular compartments is important in order to have a greater knowledge of the biogenesis, structure, and function of the different cellular organelles and their molecules. These abilities are also relevant to understand cell fate, maintenance, as well as to define the different roads of transport of proteins, lipids and other compounds in the cell.

The compounds now disclosed are highly stable and permeable, in particular the compounds are stable as a powder, in particular at room temperature, for more than a year, while other compounds like the Nile Red, for example, are stable if kept at -20 °C. Furthermore, the compounds now disclosed enter rapidly within the cells. Thus these compounds may be detected from short times, in particular 15 minutes, until several hours, in particular at least 24h. Furthermore, these compounds are responsible for subcellular staining, accumulating in organelles such as the Golgi apparatus, the nucleus, intracellular vesicles, and lipid-enriched regions/organelles such as lipid droplets, and present very low toxicity, in particular a 24 hour treatment of live cells with the compounds now disclosed do not overall affect cell viability.

This disclosure also relates to the synthesis of the above-mentioned fluorophores and their use as probes for labelling, imaging and characterizing biological samples.

These chromophores, whose synthesis is also herein disclosed, present good absorption and emission properties in the visible range, especially a large Stokes' shift. This particularity makes the separation between the exciting light and the emission easier, increasing the ratio signal/noise for a better quality of the images. The compounds are generally not cytotoxic to cells and selectively accumulate in organelles, such as the nucleus and the Golgi apparatus. These fluorophores can be used for labelling and observing these organelles and to image fixed and living cells and small organisms. Further, since their emission wavelength is red-shifted with increased solvent polarity, the disclosure can also be applied in comparative analyses of lipid-enriched cells, regions/organelles and subcellular relative polarities.

This disclosure also relates to the use of the compounds now disclosed, or any of its modifications, for emission of light.

In an embodiment, the compounds now disclosed, or any of its modifications, may be used for *in vitro* detection, identification, imaging, analysis and characterization of biological and non-biological samples, including but not limited to entire organisms, organs, tissues, cells, subcellular organelles, membranes.

In an embodiment, the compounds now disclosed, or any of its modifications, may be used for *in vivo* detection, identification, imaging, analysis and characterization of biological and non-biological samples, including molecules such as lipids.

The present disclosure has the following advantages and applications. It may be used to stain lipid droplets (LD) at low concentration, in particular 1-10×10⁻⁶ M, and through an easy experimental protocol. The disclosure is used at the same working concentrations ranges, in particular 1-10×10⁻⁶ M as Nile red, BODIPY and DHP. It stains LD very specifically when these are present in the cell. DHP working solution is also very difficult to prepare. Furthermore, the present disclosure also has solvatochromism with the polarity of the environment, similar to Nile Red, but in an opposite direction. While Nile Red shifts to the right (red emission) with the hydrophobicity of the environment, this disclosure is unique in its opposite variation: shifts to left (blue) with the increase of hydrophobicity, and to red when the environment is more polar.

The compounds now disclosed also present low cytotoxicity, in particular low cytotoxicity to mammalian cells in culture, as well as for zebrafish embryos, and thus can be used as a 'vital stain' to stain and monitor cells, and organelles, LDs and lipid membranes and molecules in live cells.

Furthermore, the disclosure may stain live cells very fast, in particular within 15 min. Usually Nile Red and DHP are used in fixed cells, and through protocols of overnight incubation.

The present disclosure is also responsible for fluorescing in the green-red range of the spectrum, colours that are easy to visualize and detect by the human eye; in contrast DHP is a blue emission fluorophore. The disclosure also presents a good intensity in cells, which increases even further in LD, having a good signal-to-noise ratio.

The present disclosure is also compatible with the worldwide used nuclear counterstaining DAPI and Hoescht dyes; DHP is not compatible with these as it mainly fluoresces in blue.

The production of the compounds now disclosed is easier, and presents good yields. In addition to being sensitive, effective and economical, the disclosure is also very stable in its powder formulation.

This disclosure may be used in scientific research, including *in vivo* cell imaging and detection and analysis of alterations in the cells' lipid profiles, and detection and analysis of LDs, and in diagnostics of diseases, syndromes and pathologies where there are lipidomic alterations, including multiple Sclerosis, chronic stress, and metabolic diseases involving lipids.

Lipid-related diseases are metabolic disorders where the synthesis, function or catabolism of lipids or lipidic molecules are impaired. This includes imbalances or abnormal production of enzymes involved in these processes that lead to an accumulation or a depletion of lipids. Other lipid-related diseases include the inability to convert stored lipids to energy. Lipid diseases are usually inherited and can cause permanent damage and even death.

The present disclosure relates to a compound, acceptable salt, ester, solvate thereof of formula 1 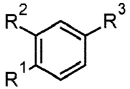 wherein
R¹ is selected from the following list: N(CH₃)₂;
R² is selected from the following list: H or halogen;
R³ is selected from the following list: 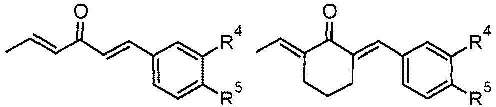
R⁴ is selected from the following list: H or halogen;
R⁵ is selected from the following list: N(CH₃)₂ or OCH₃ and
R¹, R², R³, R⁴, R⁵ are independently selected;
for use in the diagnostics of chronic stress, a lipid-related metabolic disease, lipid-related metabolic disorder, a demyelinating disease or demyelinating disorder;
wherein the demyelinating disease or demyelinating disorder is selected from the following list: multiple sclerosis, acute disseminated encephalomyelitis, Balo's disease, HTLV-I associated myelopathy, neuromyelitis optica, Schilder's disease, transverse myelitis, or cobalamin-deficiencies.

The present disclosure also relates to the compound of the present disclosure for use in the diagnostics of chronic stress, a lipid-related metabolic disease, lipid-related metabolic disorder.

In an embodiment, the demyelinating disease or demyelinating disorder is selected from the following list: multiple sclerosis, acute disseminated encephalomyelitis, Balo's disease, HTLV-1 associated myelopathy, neuromyelitis optica, Schilder's disease, transverse myelitis, or cobalamin-deficiencies.

In an embodiment, the lipid-related metabolic disease or lipid-related metabolic disorder is selected from the following list: Gaucher's disease, Tay-Sachs disease, Niemann-Pick disease, Fabry's disease, fatty acid oxidation disorders, abetalipoproteinemia, apolipoprotein B deficiency, chylomicron retention disease, familial dysbetalipoproteinemia, familial hypercholesterolemia, familial hypertriglyceridemia, inborn error of lipid metabolism, hypoalphalipoproteinemia, hypobetalipoproteinemia, lipomatosis, hypertriglyceridemia, hypercholesterolemia, hypolipoproteinemia, lecithin cholesterol acyltransferase deficiency, lipoprotein lipase deficiency, Tangier disease, Zellweger spectrum disorder, adrenoleukodystrophy, Refsum disease or Urbach-Wiethe disease.

In an embodiment, R¹ is N(CH₃)₂.

In an embodiment, R² is H, Br or Cl.

In an embodiment, R⁴ is H, Br or Cl.

In an embodiment, R⁵ is N(CH₃)₂.

In an embodiment, R¹ is N(CH₃)₂.

In an embodiment for better results the compound may be (1E,4E)-1,5-bis[3-chloro-4-(dimethylamino)phenyl]penta-1,4-dien-3-one.

In an embodiment for better results the compound may be (1E,4E)-1,5-bis[3-bromo-4-(dimethylamino)phenyl]penta-1,4-dien-3-one.

In an embodiment for better results the compound may be (2E,6E)-2,6-bis[4-(dimethylamino)benzylidene]cyclohexanone.

In an embodiment for better results the compound may be (2E,6E)-2,6-bis[3-chloro-4-(dimethylamino)benzylidene]cyclohexanone.

In an embodiment for better results the compound may be (2E,6E)-2,6-bis[3-bromo-4-(dimethylamino)benzylidene]cyclohexanone.

In an embodiment, any of the compounds now disclosed may be used for diagnostics of diseases where the lipidic spectrum is altered, including chronic stress, lipid-related metabolic diseases or lipid-related metabolic disorders, or a demyelinating disease or a demyelinating disorder, namely in the treatment of a demyelinating disease or demyelinating disorder selected from the following list: multiple sclerosis, acute disseminated encephalomyelitis, Balo's disease, HTLV-I associated myelopathy, neuromyelitis optica, Schilder's disease, transverse myelitis, or disorders related to cobalamin-deficiencies.

In an embodiment, any of the compounds now disclosed may be used for diagnostics of chronic stress, lipid-related metabolic disease or lipid-related metabolic disorder or a demyelinating disease or a demyelinating disorder, namely in the diagnostics of a lipid-related metabolic disease or lipid-related metabolic disorder selected from the following list: Gaucher's disease, Tay-Sachs disease, Niemann-Pick disease, Fabry's disease, fatty acid oxidation disorders, abetalipoproteinemia, apolipoprotein B deficiency, chylomicron retention disease, familial dysbetalipoproteinemia, familial hypercholesterolemia, familial hypertriglyceridemia, inborn error of lipid metabolism, hypoalphalipoproteinemia, hypobetalipoproteinemia, lipomatosis, hypertriglyceridemia, hypercholesterolemia, hypolipoproteinemia, lecithin cholesterol acyltransferase deficiency, lipoprotein lipase deficiency, Tangier disease, Zellweger spectrum disorder, adrenoleukodystrophy, Refsum disease or Urbach-Wiethe disease.

Throughout the description and claims the word "comprise" and variations of the word, are not intended to exclude other technical features, additives, components, or steps. Additional objectives, advantages and features of the solution will become apparent to those skilled in the art upon examination of the description or may be learned by practice of the solution.

### Brief description of the drawings

The following figures provide preferred embodiments for the present disclosure and should not be seen as limiting the scope of the disclosure.

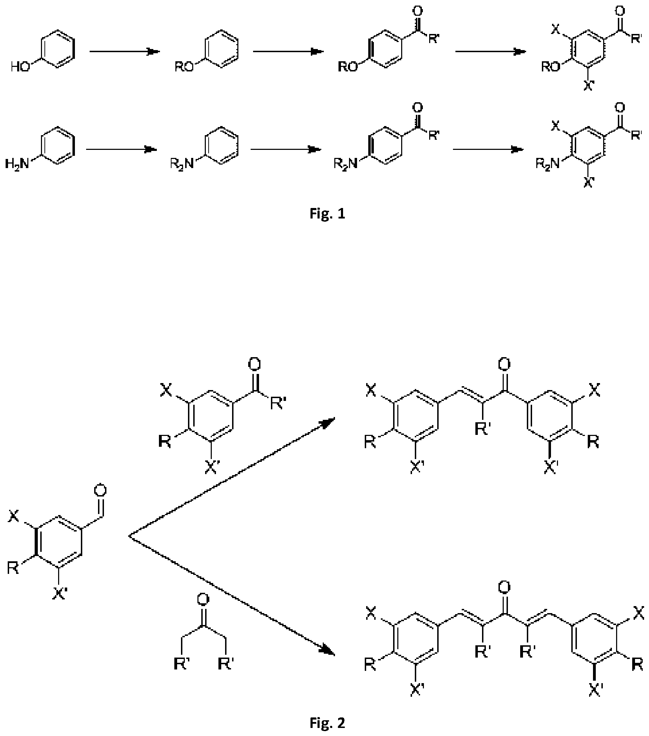

**Figure 1****:** Scheme of the fluorophore precursors synthesis.
**Figure 2****:** Synthesis of the fluorophores.

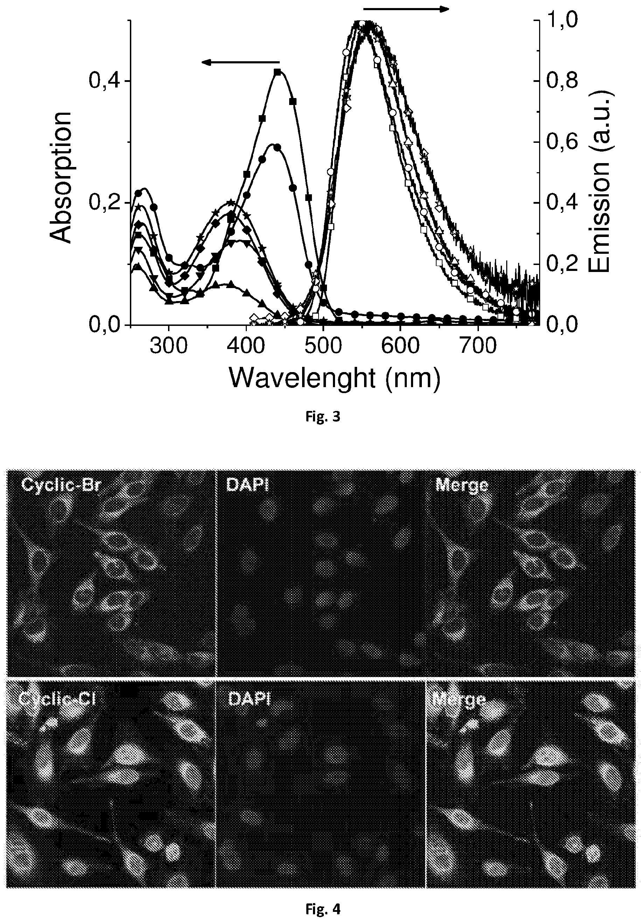

**Figure 3****:** Absorption and emission spectra of some of the fluorophores in a dichloromethane (DCM) solution wherein black squares represent the absorption of (1*E,*4*E*)-1,5-bis[4-(dimethylamino)phenyl]penta-1,4-dien-3-one; black circles represent the absorption of (2*E*,6*E*)-2,6-bis[4-(dimethylamino)benzylidene]cyclohexanone; black diamonds represent the absorption of 2,6-bis[3-bromo-4-(dimethylamino)benzylidene]cyclohexanone; black triangles pointing up represent the absorption of (1*E*,4*E*)-1,5-bis[3-bromo-4-(dimethylamino)phenyl]penta-1,4-dien-3-one; black triangles pointing down represent the absorption of (1*E*,4*E*)-1,5-bis[3-chloro-4-(dimethylamino)phenyl]penta-1,4-dien-3-one; black stars represent the absorption of 2,6-bis[3-chloro-4-(dimethylamino)benzylidene]cyclohexanone; open squares represent the emission of (1*E,*4*E*)-1,5-bis[4-(dimethylamino)phenyl]penta-1,4-dien-3-one; open circles represent the emission of (2*E*,6*E*)-2,6-bis[4-(dimethylamino)benzylidene]cyclohexanone; open diamonds represent the emission of 2,6-bis[3-bromo-4-(dimethylamino)benzylidene]cyclohexanone; open triangles pointing up represent the absorption of (1*E*,4*E*)-1,5-bis[3-bromo-4-(dimethylamino)phenyl]penta-1,4-dien-3-one; open triangles pointing down represent the absorption of (1*E*,4*E*)-1,5-bis[3-chloro-4-(dimethylamino)phenyl]penta-1,4-dien-3-one; open stars represent the absorption of 2,6-bis[3-chloro-4-(dimethylamino)benzylidene]cyclohexanone.
**Figure 4****:** Sample distributions of two cyclic fluorophores: (2*E*,6*E*)-2,6-bis[3-bromo-4-(dimethylamino)benzylidene]cyclohexanone labelled as cyclic-Br and (2*E*,6*E*)-2,6-bis[3-chloro-4-(dimethylamino)benzylidene]cyclohexanone labelled as cyclic-Cl (orange pseudocolour) in HeLa cells cultured on coverslips and incubated alive and for 1h with 10⁻⁵ mol.L⁻¹ of the compounds. The laser line 458 nm was used to excite and the confocal Zeiss LSM-510 META 415 to 750 nm detector used to detect the fluorophores. The nuclear DAPI is used as a counterstaining dye.

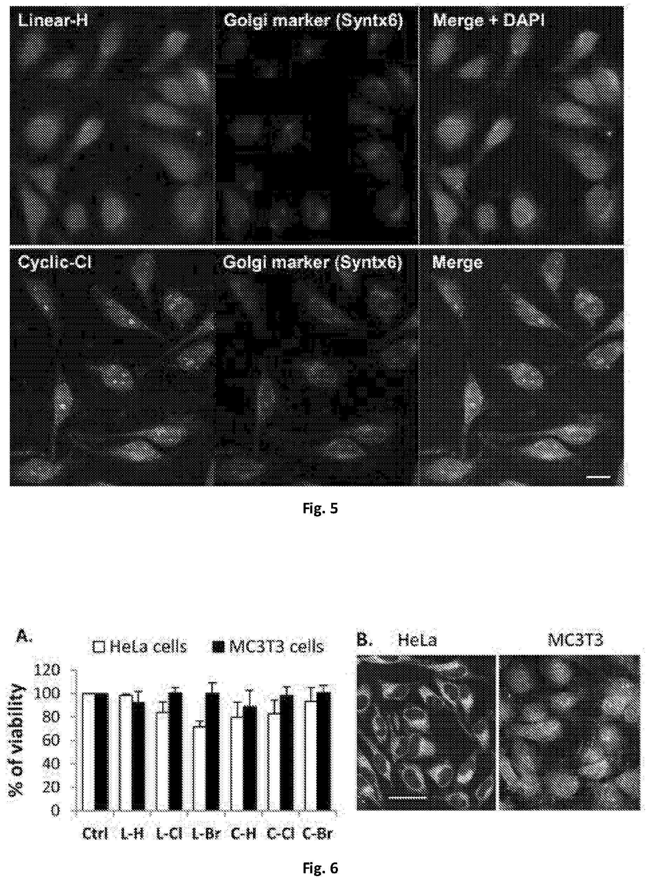

**Figure 5****:** Co-localization of the compounds (1*E*,4*E*)-1,5-bis[4-(dimethylamino)phenyl]penta-1,4-dien-3-one, labelled as linear-H and (2*E*,6*E*)-2,6-bis[3-chloro-4-(dimethylamino)benzylidene]cyclohexanone, labelled as cyclic-Cl with the Trans-Golgi Network (TGN) marker Syntaxin-6, which was Texas Red (red colour) and Alexa-405 (cyan pseudocolour) labelled by using secondary antibodies. DAPI (blue colour) was used as nuclear marker. HeLa cells were incubated in particular for 1h with 1-2×10⁻⁵ mol.L⁻¹ of the fluorophores, before being fixed. The laser line 405 nm, in particular at 10-20% of its power was used to excite the fluorophores. Bar, 10 µm.
**Figure 6****:** Shows rat MC3T3-E1 and human HeLa cells viabilities following their exposition to the fluorophores for 24h. **A.** Cell viability was determined using the metabolic Resazurin assay, and data (resazurin final O.D.±SD of four independent experiments) presented graphically as percentages of control cells (not incubated with the compounds), wherein the control is labelled as Ctrl; (1*E*,4*E*)-1,5-bis[4-(dimethylamino)phenyl]penta-1,4-dien-3-one is labelled as L-H; (1*E*,4*E*)-1,5-bis[3-chloro-4-(dimethylamino)phenyl]penta-1,4-dien-3-one is labelled as L-Cl; (1*E*,4*E*)-1,5-bis[3-bromo-4-(dimethylamino)phenyl]penta-1,4-dien-3-one is labelled as L-Br; (2*E*,6*E*)-2,6-bis[4-(dimethylamino)benzylidene]cyclohexanone labelled is labelled as C-H; 2,6-bis[3-chloro-4-(dimethylamino)benzylidene]cyclohexanone is labelled as C-Cl; 2,6-bis[3-bromo-4-(dimethylamino)benzylidene]cyclohexanone is labelled as C-Br. Statistical significance using the student-t test: *p<0.05; **p<0.01. **B.** Microphotograph of Cyclic-Cl distribution in HeLa and MC3T3 cells upon 24h of exposure to the compounds. Microscope settings used: a 40×/1.3 oil objective; HFT 405/488/633; in particular a 405 nm laser line at 40% of its power. Bar, 50 µm.

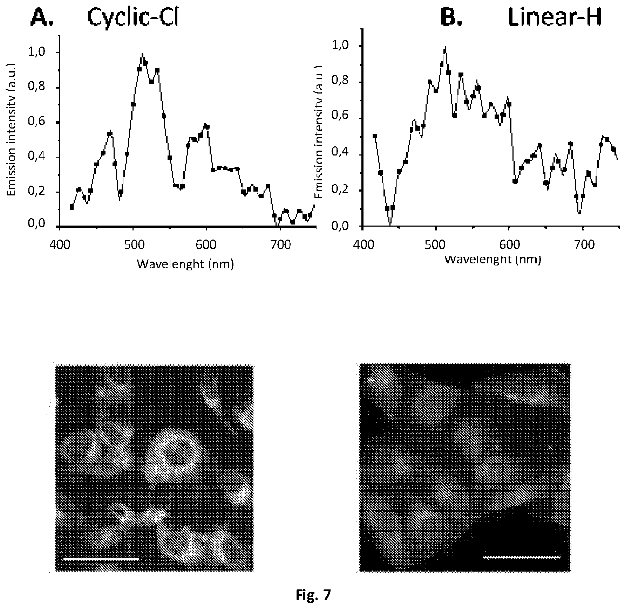

**Figure 7****:** Emission lambda scans of two of the fluorophores((1*E*,4*E*)-1,5-bis[4-(dimethylamino)phenyl]penta-1,4-dien-3-one, labelled as linear-H and (2*E*,6*E*)-2,6-bis[3-chloro-4-(dimethylamino)benzylidene]cyclohexanone, labelled as cyclic-Cl, excited at 405 nm of excitation, in particular with the laser at 15% of its power. HeLa cells were incubated for 1 hour with 2-10 ×10⁻⁵ mol.L⁻¹ of the fluorophores, before being fixed. Below: confocal microphotographs of the cells with their respective lambda colours. Bar, 20 µm.

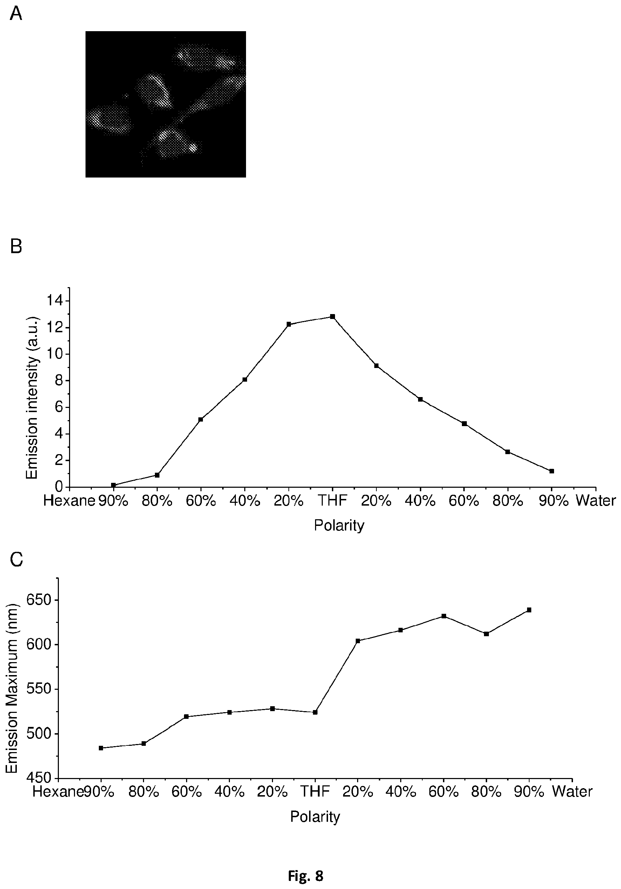

**Figure 8****:** Confocal microphotograph of HeLa cells enriched in lipid droplets and stained with (2*E*,6*E*)-2,6-bis[3-chloro-4-(dimethylamino)benzylidene]cyclohexanone (cyclic-Cl) showing the shift to the left (green lambda colour) in a lipid-enriched less polar cellular compartment **(A);** variation of the (1*E*,4*E*)-1,5-bis[4-(dimethylamino)phenyl]penta-1,4-dien-3-one (linear-H) emission maximum **(B)** and intensity **(C)** versus solvent polarity (example for ((1*E*,4E)-1,5-bis[4-(dimethylamino)phenyl]penta-1,4-dien-3-one).

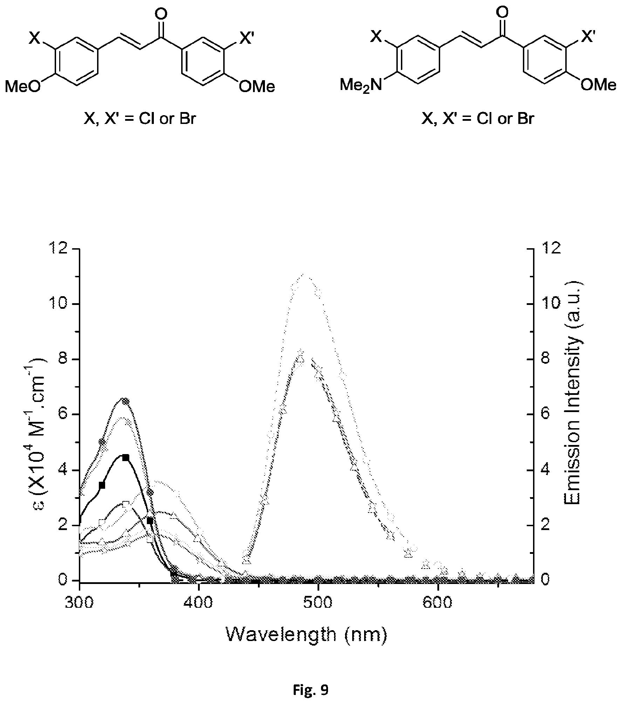

**Figure 9****:** Absorption and emission spectra of some of the fluorophores in a tetrahydrofuran (THF) solution wherein full lines represent the absorption and dotted lines the emission; and wherein black squares represent (*E*)-1,3-bis(3-chloro-4-methoxyphenyl)prop-2-en-1-one; black circles represent (*E*)-1-(3-bromo-4-methoxyphenyl)-3-(3-chloro-4-methoxyphenyl)prop-2-en-1-one; open squares represent (*E*)-1,3-bis(3-bromo-4-methoxyphenyl)prop-2-en-1-one; grey triangles represent (*E*)-3-(3-bromo-4-methoxyphenyl)-1-(3-chloro-4-methoxyphenyl)prop-2-en-1-one; open circles represent (*E*)-3-(3-chloro-4-(dimethylamino)phenyl)-1-(3-chloro-4-methoxyphenyl)prop-2-en-1-one; open stars represent (*E*)-3-(3-bromo-4-(dimethylamino)phenyl)-1-(3-bromo-4-methoxyphenyl)prop-2-en-1-one; open triangles represent (*E*)-1-(3-bromo-4-methoxyphenyl)-3-(3-chloro-4-(dimethylamino)phenyl)prop-2-en-1-one.

### Detailed description

The present disclosure relates a compound for use in the diagnostics of chronic stress, a lipid-related metabolic disease, lipid-related metabolic disorder, a demyelinating disease or demyelinating disorder.

The particular stability of the powder formulations of the fluorophores of the present disclosure, their overall non-cytotoxicity, and their fluorescent properties, strengthen their use in cell imaging, including live cell imaging.

One aspect of the present disclosure relates to a compound of formula (2), wherein X is H, Cl or Br, and the ketone is cyclic or linear. These have been synthesized in large quantities, in particular on multiple grams scale, following a common strategy. The building blocks were obtained by chloration or bromation of 4-dimethylaminobenzaldehyde using N-Chlorosuccinimide or N-Bromosuccinimide **(****Fig. 1****).** The double condensation of the appropriate benzaldehyde derivative with acetone or cyclohexanone gave the linear and cyclic chromophores, respectively, with good yields **(****Fig. 2****).**

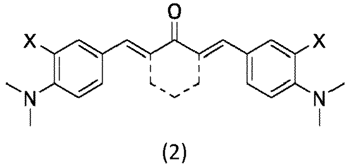

In an embodiment, the compounds now disclosed present a good quantum yield in solution, and a large Stokes' shift **(****Fig. 3****).** The large Stokes' shift observed for all compounds is characteristic of a transition having charge transfer character, which would be from the dimethylamino group to the ketone via the two butadiene bridges. Their powder formulation is stable, in particular for more than one year, and the compounds can be used as a solution in DMSO or ethanol.

In an embodiment, live HeLa cells were incubated for one hour with 1-2×10⁻⁵ mol.L⁻¹ of the compounds, which are internalized and fluoresce well inside cells, as detected by confocal microscopy **(****Fig. 4****).** Fluorescence exhibits a punctate distribution, and was mainly detected in the membranar subcellular localizations, and not in the aqueous cytoplasm or outside the cell. The H substitution by a chloride resulted in a fluorophore with brighter emission when inside cells **(****Fig. 4** and **5****).**

In an embodiment, the compounds (1*E*,4*E*)-1,5-bis[4-(dimethylamino)phenyl]penta-1,4-dien-3-one (linear-H) and (2*E*,6*E*)-2,6-bis[3-chloro-4-(dimethylamino)benzylidene]cyclohexanone (cyclic-Cl) initially localize more abundantly at the Golgi region and further on also at the nucleus, a distribution confirmed by co-localization with the Trans-Golgi protein marker Syntaxin-6 and the nuclear DAPI dye **(****Fig. 4** and **5****).** Their enriched Golgi and nuclei localization make the compounds promising fluorescing probes for organelle detection, as BODIPY chromophores.

In an embodiment, the compounds now disclosed are not cytotoxic to cells, as **Fig. 6** discloses, and selectively accumulate in organelles, such as the nucleus and the Golgi apparatus **(****Fig. 3** and **4****).** These fluorophores can be used for labelling and observing these organelles and to image fixed and living cells **(****Fig. 7****).** Further, since their emission wavelength is red-shifted with increased solvent polarity, the disclosure can also be applied in comparative analyses of lipid-enriched regions/organelles and subcellular relative polarities **(****Fig. 8****).**

In an embodiment, the compounds now disclosed also present low cytotoxicity, in particular low cytotoxicity to mammalian cells in culture, as well as zebrafish embryos, and thus can be used to stain live cells and monitor LDs, lipidic membranes and molecules in live cells, in particular as a 'vital stain' **(****Fig. 6****).**

In an embodiment, cells fluoresced either in bright green, yellow and orange, depending on the excitation and emission filter sets chosen, but almost nothing in blue.

In an embodiment, the compounds are not cytotoxic to cells even following 24h of exposition **(****Fig. 6A****).** Human HeLa and rat MC3T3 live cells were incubated for 24h with 1×10⁻⁵ mol.L⁻¹ of the compounds (1*E*,4*E*)-1,5-bis[4-(dimethylamino)phenyl]penta-1,4-dien-3-one (linear-H), (1*E*,4*E*)-1,5-bis[3-chloro-4-(dimethylamino)phenyl]penta-1,4-dien-3-one (linear-Cl), (1*E*,4*E*)-1,5-bis[3-bromo-4-(dimethylamino)phenyl]penta-1,4-dien-3-one (linear-Br), (2*E*,6*E*)-2,6-bis[4-(dimethylamino)benzylidene]cyclohexanone (cyclic-H), (2*E*,6*E*)-2,6-bis[3-chloro-4-(dimethylamino)benzylidene]cyclohexanone (cyclic-Cl), (2*E*,6*E*)-2,6-bis[3-bromo-4-(dimethylamino)benzylidene]cyclohexanone (cyclic-Br). In the last 4h of the 24h exposure period, cells were incubated with fresh medium containing 10% of a resazurin solution. The media was spectrophotometrically measured to calculate the extent of resazurin reduction to resofurin by viable cells. Results are presented as percentages of control cell viability, meaning not incubated with the tested compounds.

In an embodiment and in accordance to their green to yellow-red emissions, the compounds emission profiles have two peaks around 525 and 590 nm or a wider band instead of these two peaks **(****Fig. 7****).** The compounds could be excited with the 405 nm laser line, even at lower potencies, or the 458 nm laser line, in particular at higher power. Lambda scans were recorded using a 405 nm diode laser as the excitation source.

In an embodiment, the compounds when inside the cells were observed not to homogenously stain the cells but instead to fluoresce differentially, in particular green, yellow, and even orange depending on their localization **(****Fig. 7** microphotographs).

In an embodiment, the fluorophores alter their emission spectra accordingly to the physic-chemical subcellular environment they are in, in particular to the solvent polarity **(****Fig. 8****).** The disclosure shifts left in its emission wavelength (greener emission colours) when the solvent is more hydrophobic (and in the highly hydrophobic lipid droplets inside cells, **Fig. 8a****)** and shifts right in its emission wavelength (more yellow-red emission colours) when the solvent is more polar.

The present disclosure has similarities with Nile Red, given that both stain living cells, are highly membrane-permeant and not acutely toxic, and their emission is virtually absent in the blue region, allowing to be used in combination with relevant blue-emitting probes such as DAPI or Hoechst. This disclosure can also detect differences in the relative hydrophobic strengths of the membranes it is in, similar to Nile Red, a known marker of lipid droplets that shifts in its fluorescence emission according to the level of hydrophobicity of its solvent.

The present disclosure may be used in multiple applications regarding cellular and subcellular visualization, imaging, detection, and characterization.

In an embodiment, a possible application concerns studies of live cell imaging, such as those of cell migration and tissue regeneration, where cell detection and visualization is required. In applications such as these, the probes now disclosed would penetrate live or dead cells, either isolated cells or cells in tissues, organs and organisms, and turn them fluorescent and hence detected by various means, as fluorescent microscopy. An example of object cell to visualize is the generally translationally silent sperm cell, where some well-known genome-altering fluorophores, such as GFP and its derivatives, cannot be used.

The compounds now disclosed have properties, such as its high permeability to live cell membranes (as high as Nile Red and BODIPY) and overall non-cytotoxicity that adequate them to various experimental setups for live cell imaging. In this, possible phototoxicity due to longer imaging with a 405 nm excitation line can be circumvented by the use of two-photon microscopy.

In an embodiment, a possible application of this disclosure is as organelle marker in various studies, including those aiming to study the biogenesis and morphology of such organelles, and those where co-localization of molecules with organelle markers are required.

In an embodiment, the compounds now disclosed may be used as Golgi-marker, in particular the fluorophores (1*E*,4*E*)-1,5-bis[4-(dimethylamino)phenyl]penta-1,4-dien-3-one (linear-H) and (2*E*,6*E*)-2,6-bis[3-chloro-4-(dimethylamino)benzylidene]cyclohexanone (cyclic-Cl) may be used as Golgi-marker. The Golgi network is a central organelle that processes all the proteins of the secretory pathway and plasma membrane. These are very valuable to study the trafficking dynamics and localization of proteins exiting and returning to the TGN, as the Alzheimer's amyloid precursor protein. These markers could also assist studies on differentiation-related Golgi proteins, as in the imaging of the TGN-abundant type-I collagen protein involved in osteoblasts differentiation.

In an embodiment, the compounds now disclosed, in particular the fluorophores (1*E*,4*E*)-1,5-bis[4-(dimethylamino)phenyl]penta-1,4-dien-3-one (linear-H) and (2*E*,6*E*)-2,6-bis[3-chloro-4-(dimethylamino)benzylidene]cyclohexanone (cyclic-Cl) may be used as a nuclear marker and assist in studies of nuclear molecules involved in various physiological and pathological cellular events, including expression, proliferation and differentiation events, which is of relevance as the nucleus is a major cellular organelle that determines cell life and fate.

In an embodiment, the compounds now disclosed, in particular the fluorophores (1*E*,4*E*)-1,5-bis[4-(dimethylamino)phenyl]penta-1,4-dien-3-one (linear-H) and (2*E*,6*E*)-2,6-bis[3-chloro-4-(dimethylamino)benzylidene]cyclohexanone (cyclic-Cl), may be used as a probe for solvent polarity, such as a lipophilic fluorescence probe.

In an embodiment, the compounds now disclosed, in particular the fluorophores (1*E*,4*E*)-1,5-bis[4-(dimethylamino)phenyl]penta-1,4-dien-3-one (linear-H) and (2*E*,6*E*)-2,6-bis[3-chloro-4-(dimethylamino)benzylidene]cyclohexanone (cyclic-Cl) can be used in *in vitro* and *in vivo* studies aiming to but not limited to estimate the level of hydrophobicity of the solvent; identify apolar organelles such as lipid droplets; compare differences in organelle and membranes polarity; detect alterations in the lipid composition of cellular membranes; detect molecules such as lipids and lipid-enriched molecules and compare their relative hydrophobic strength.

In an embodiment, since the emission shift occurs to the right with increased polarity, the disclosure would be more greenish in organelles and membranes more hydrophobic, and more yellow-to-red in more polar ones.

In an embodiment, the compounds now disclosed may be used in fluorescence-related techniques that include but are not limited to fluorescence spectroscopy; fluorescence microscopy; fluorescence imaging; fluorescence labelling; ground state depletion microscopy; single-molecule spectroscopy; Forster resonance energy transfer applications; fluorescence correlation spectroscopy; fluorescence anisotropy spectroscopy; correlative fluorescence-electron microscopy; fluorescence activated cell sorting, cytometry and other related techniques such as image streaming.

Examples of probes now disclosed:

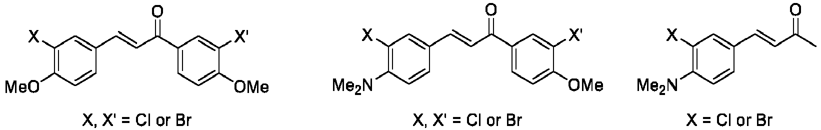

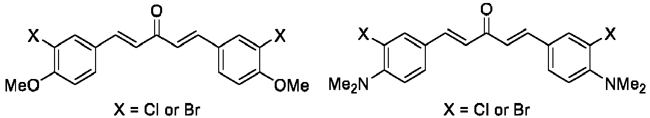

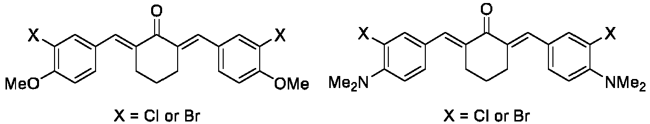

The synthesis of the probes is now described.

The synthesis of (1*E*,4*E*)-1,5-bis[4-(dimethylamino)phenyl]penta-1,4-dien-3-one - example 1, (1*E*,4*E*)-1,5-bis[3-chloro-4-(dimethylamino)phenyl]penta-1,4-dien-3-one - example 2, (1*E*,4*E*)-1,5-bis[3-bromo-4-(dimethylamino)phenyl]penta-1,4-dien-3-one - example 3, (2*E*,6*E*)-2,6-bis[4-(dimethylamino)benzylidene]cyclohexanone - example 4, (2*E*,6*E*)-2,6-bis[3-chloro-4-(dimethylamino)benzylidene]cyclohexanone - example 5, (2*E*,6*E*)-2,6-bis[3-bromo-4-(dimethylamino)benzylidene]cyclohexanone - example 6 comprises the following steps: 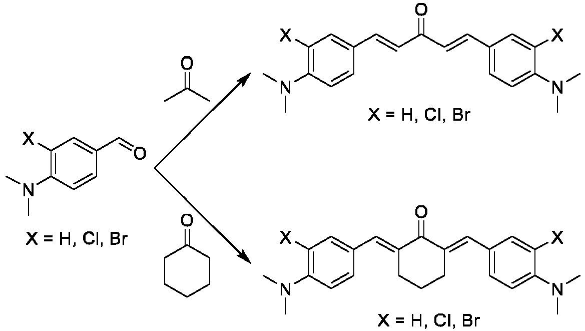 adding of sodium hydroxide, in particular 2 equivalents, to a solution of the benzaldehyde derivative, in particular 2 equivalents, and a ketone, in particular 1 equivalent, in absolute ethanol;
stirring the resulting solution at room temperature, in particular 20-25 °C for 2-4 h;
evaporating the ethanol;
purifying the obtained probe by silica gel flash column chromatography with an eluent, in particular CH₂Cl₂/MeOH, 95/5.

The characterization of the compounds (1*E*,4*E*)-1,5-bis[4-(dimethylamino)phenyl]penta-1,4-dien-3-one - example 1, (1*E*,4*E*)-1,5-bis[3-chloro-4-(dimethylamino)phenyl]penta-1,4-dien-3-one - example 2, (1*E*,4*E*)-1,5-bis[3-bromo-4-(dimethylamino)phenyl]penta-1,4-dien-3-one - example 3, (2*E*,6*E*)-2,6-bis[4-(dimethylamino)benzylidene]cyclohexanone - example 4, (2*E,*6*E*)-2,6-bis[3-chloro-4-(dimethylamino)benzylidene]cyclohexanone - example 5, (2*E*,6*E*)-2,6-bis[3-bromo-4-(dimethylamino)benzylidene]cyclohexanone - example 6 is now disclosed.

### (1E,4E)-1,5-bis[4-(dimethylamino)phenyl]penta-1,4-dien-3-one - Example 1

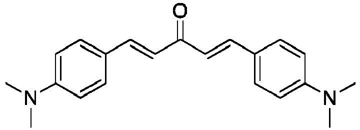

In an embodiment, the characterization of the (1E,4E)-1,5-bis[4-(dimethylamino)phenyl]penta-1,4-dien-3-one compound is as follows: a red solid product was obtained, with a 31% yield, a melting point (m.p.) of 193-195 °C (lit. 185 °C). Further ¹H NMR (300.13 MHz, CDCl₃, 25 °C): δ 7.69 (d, ³*J*_{H-H} 15.7 Hz, 2H, H-β), 7.52 (d, ³*J*_{H-H} 9.0 Hz, 4H, H-2,6), 6.89 (d, ³*J*_{H-H} 15.7 Hz, 2H, H-α), 6.69 (d, ³*J*_{H-H} 9.0 Hz, 4H, H-3,5), 3.04 (s, 12H, C*H*₃).

### (1E,4E)-1,5-bis[3-chloro-4-(dimethylamino)phenyl]penta-1,4-dien-3-one - Example 2

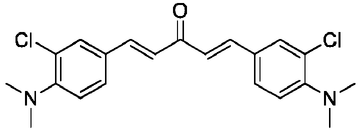

In an embodiment, the characterization of the (1E,4E)-1,5-bis[3-chloro-4-(dimethylamino)phenyl]penta-1,4-dien-3-one compound is as follows: an orange oil, with a yield of 57%. Further ¹H NMR (300.13 MHz, CDCl₃, 25 °C): δ 7.63 (d, ⁴*J*_{H-H} 2.1 Hz, 2H, H-2), 7.62 (d, ³*J*_{H-H} 15.8 Hz, 2H, H-β), 7.43 (dd, ⁴*J*_{H-H} 2.1, 3*J*_{H-H} 8.4 Hz, 2H, H-6), 7.03 (d, ³*J*H-H 8.4 Hz, 2H, H-5), 6.95 (d, ³*JH-H* 15.8 Hz, 2H, H-α), 2.89 (s, 12H, CH₃). ¹³C NMR (75 MHz, CDCl₃, 25 °C): δ 188.3 (C=O), 152.1 (C-N), 141.6 (C=C), 130.4 (C-H), 129.1 (Cquat), 128.0 (C-H), 127.4 (Cquat), 124.2 (C-H), 119.6 (C=C), 43.3 (CH₃). TOF HRMS ESI Calcd. for C₂₁H₂₂N₂O₁Cl₂: 388.1109 (100 %), 390.1080 (65 %). Found: 388.1103 (100 %) (-1.5 ppm), 390.1086 (64%) (+1.5 ppm).

### (1E,4E)-1,5-bis[3-bromo-4-(dimethylamino)phenyl]penta-1,4-dien-3-one - Example 3

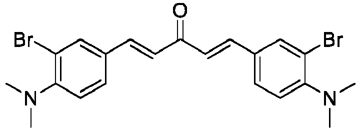

In an embodiment, the characterization of the (1*E*,4*E*)-1,5-bis[3-bromo-4-(dimethylamino)phenyl]penta-1,4-dien-3-one compound is as follows: orange solid, 44% yield and m.p. 94-96 °C. Further ¹H NMR (300.13 MHz, CDCl₃, 25 °C): δ 7.83 (d, ⁴J_{H-H} 2.1 Hz, 2H, aromatic H-2), 7.62 (d, ³JH-H 15.9 Hz, 2H, H-β), 7.48 (dd, ⁴*J*_{H-H} 2.1, ³*J*_{H-H} 8.4 Hz, 2H, H-6), 7.04 (d, ³*J*_{H-H} 8.4 Hz, 2H, H-5), 6.95 (d, ³*J*_{H-H} 15.9 Hz, 2H, H-α), 2.88 (s, 12H, CH₃). ¹³C NMR (75 MHz, CDCl₃, 25 °C): δ 188.3 (C=O), 153.6 (C-N), 141.5 (C=C), 133.7 (C-H), 129.9 (Cquat), 128.6 (C-H), 124.4 (Cquat), 120.1 (C-H), 118.1 (C=C), 43.8 (CH₃). TOF HRMS ESI Calcd. for C₂₁H₂₂N₂O₁Br₂: 476.0099 (50 %), 478.0078 (100 %), 480.0058 (50 %). Found: 476.0103 (45 %) (+0.4 ppm), 478.0080 (100 %) (+0.4 ppm), 480.0066 (49 %) (+1.7 ppm).

### (2E,6E)-2,6-Bis[4-(dimethylamino)benzylidene]cyclohexanone - Example 4

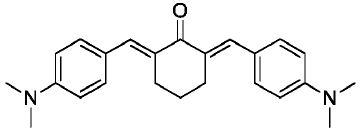

In an embodiment, the characterization of the (2*E*,6*E*)-2,6-bis[4-(dimethylamino)benzylidene]cyclohexanone compound is as follows red solid, 95% yield and a m.p. 239-241 °C (lit. 160 °C). Further ¹H NMR (300.13 MHz, CDCl₃, 25 °C): δ 7.76 (br s, 2H, C=C*H*), 7.44 (d, ³*J*_{H-H} 8.9 Hz, 4H, H-2',6'), 6.72 (d, ³*J*_{H-H} 8.9 Hz, 4H, H-3',5'), 3.02 (s, 12H, C*H*₃), 3.02-2.91 (m, 4H, C*H*₂), 1.85-1.77 (m, 2H, C*H*₂).

### (2E,6E)-2,6-Bis[3-chloro-4-(dimethylamino)benzylidene]cyclohexanone - Example 5

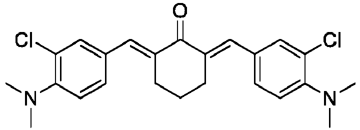

In an embodiment, the characterization of the (2*E*,6*E*)-2,6-bis[3-chloro-4-(dimethylamino)benzylidene]cyclohexanone compound was obtained as an orange solid, 75% yield and a m.p. of 130-132 °C. Further ¹H NMR (300.13 MHz, CDCl₃, 25 °C): δ 7.68 (bs, 2H, C=CH), 7.50 (d, ⁴*J*_{H-H} 2.0 Hz, 2H, H-2'), 7.33 (dd, ⁴*J*_{H-H} 2.0, ³*J*_{H-H} 8.4 Hz, 2H, H-6'), 7.05 (d, ³*J*_{H-H} 8.4 Hz, 2H, H-5'), 2.93-2.87 (m, 4H, CH₂), 2.88 (s, 12H, CH₃), 1.85-1.77 (m, 2H, CH₂). ¹³C NMR (75 MHz, CDCl₃, 25 °C): δ 189.7 (C=O), 150.5 (C-N), 135.5 (C=C), 135.2 (Cquat), 132.6 (C-H), 130.6 (Cquat), 130.1 (C-H), 127.2 (C-H), 119.3 (C=C), 43.4 (CH₃), 28.4 (CH₂), 22.8 (CH₂). ESI-MS: m/z: 429.2 (100 %), 431.2 (60%) [M+H]+, 215.1 (80%), 216.1 (50%) [M+2H]2+. Anal. Calcd. for C₂₄H₂₆Cl₂N₂O: C, 67.13; H, 6.10; N, 6.52. Found: C, 67.26; H, 6.16, N, 6.23.

### (2E,6E)-2,6-Bis[3-bromo-4-(dimethylamino)benzylidene]cyclohexanone - Example 6

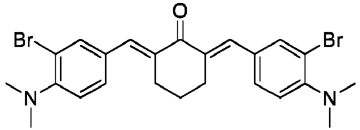

In an embodiment, the characterization of the (2*E*,6*E*)-2,6-bis[3-bromo-4-(dimethylamino)benzylidene]cyclohexanone was obtained as an orange solid, 48% yield and a m.p. 154-156 °C. Further ¹H NMR (300.13 MHz, CDCl₃, 25 °C): δ 7.69 (d, ⁴*J*_{H-H} 2.0 Hz, 2H, H-2'), 7.67 (br s, 2H, C=CH), 7.38 (dd, ⁴*J*_{H-H} 2.0, ³*J*_{H-H} 8.4 Hz, 2H, H-6'), 7.07 (d, ³*J*_{H-H} 8.4 Hz, 2H, H-5'), 2.93-2.86 (m, 4H, CH₂), 2.86 (s, 12H, CH₃), 1.85-1.79 (m, 2H, CH2). ¹³C NMR (75 MHz, CDCl₃, 25 °C): δ 189.7 (C=O), 152.0 (C-N), 135.8 (C=C), 135.3 (Cquat), 135.2 (Cquat), 131.3 (C-H), 130.6 (C-H), 119.8 (C=C), 118.0 (C-H), 43.9 (CH₃), 28.4 (CH₂), 22.8 (CH₂). ESI-MS: m/z: 517.1 (40 %), 519.1 (100 %), 521.1 (40 %) [M+H]+, 259.0 (50 %), 260.0 (100 %), 261.0 (50 %) [M+2H]2+. Anal. Calcd for C₂₄H₂₆Br₂N₂O: C 55.62, H 5.06, N 5.41. Found: C 55.62, H 4.99, N 5.19 %.

The synthesis and characterization of (*E*)-1,3-bis(3-chloro-4-methoxyphenyl)prop-2-en-1-one - example 7, (*E*)-1-(3-bromo-4-methoxyphenyl)-3-(3-chloro-4-methoxyphenyl)prop-2-en-1-one - example 8, (*E*)-3-(3-bromo-4-methoxyphenyl)-1-(3-chloro-4-methoxyphenyl)prop-2-en-1-one - example 9, (*E*)-1,3-bis(3-bromo-4-methoxyphenyl)prop-2-en-1-one - example 10, (*E*)-3-(3-chloro-4-(dimethylamino)phenyl)-1-(3-chloro-4-methoxyphenyl)prop-2-en-1-one - example 11, (*E*)-1-(3-bromo-4-methoxyphenyl)-3-(3-chloro-4-(dimethylamino)phenyl)prop-2-en-1-one - example 12, (*E*)-3-(3-bromo-4-(dimethylamino)phenyl)-1-(3-chloro-4-methoxyphenyl)prop-2-en-1-one - example 13, (*E*)-3-(3-bromo-4-(dimethylamino)phenyl)-1-(3-bromo-4-methoxyphenyl)prop-2-en-1-one - example 14 are now disclosed.

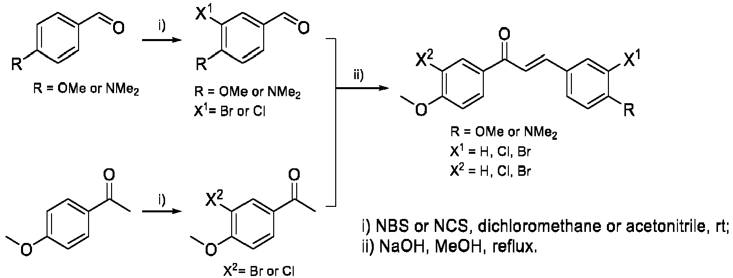

### (E)-1,3-bis(3-chloro-4-methoxyphenyl)prop-2-en-1-one - Example 7

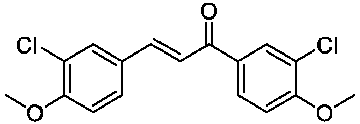

In an embodiment, 3-chloro-4-methoxyacetophenone, in particular 50 mg, 0.27 mmol, 1 equiv., and 3-chloro-4-methoxybenzaldehyde, in particular 70 mg, 0.41 mmol, 1.5 equiv., were dissolved in methanol (MeOH), in particular were dissolved in 10 mL of MeOH. NaOH, in particular 43 mg, 1.08 mmol, 4 equiv., was added slowly. The reaction mixture was stirred under reflux during 4 h, cooled to room temperature, poured into ice and acidified with dilute HCl. The mixture was extracted with dichloromethane, washed with water, dried over sodium sulphate, and evaporated. The residue was purified by chromatography, in particular dichloromethane/hexane: 2/1, by TLC to give the product (*E*)-1,3-bis(3-chloro-4-methoxyphenyl)prop-2-en-1-one as a yellow solid, in particular 76 mg of the (*E*)-1,3-bis(3-chloro-4-methoxyphenyl)prop-2-en-1-one with a yield of 85%.

In an embodiment, the characterization of (*E*)-1,3-bis(3-chloro-4-methoxyphenyl)prop-2-en-1-one is as follows: yellow solid, m.p. of 164-165 °C, ESI-MS: m/z: 337.0 [M ³⁵Cl ³⁵Cl +H]⁺ (100%), 339.1 [M ³⁵Cl ³⁷Cl +H]⁺ (60%), 341.0 [M ³⁷Cl³⁷Cl +H]⁺ (10%). Anal Calcd (%) for C₁₇H₁₄Cl₂O₃ .H₂O (355.21): C 57.48, H 4.54; found C 57.41, H 4.23; ¹H NMR (300 MHz, CDCl₃, 25 °C): δ = 8.09 (d, ⁴*J*_{H-H} 2.1 Hz, 1H, H aromatic), 7.97 (dd, ⁴*J*_{H-H} 2.1, ³*J*_{H-H} 8.6 Hz, 1H, H aromatic), 7.72 (d, ³*J*_{H-H} 15.5 Hz, 1H, CH=CH), 7.71 (d, ⁴*J*_{H-H} 2.1 Hz, 1H, H aromatic), 7.50 (dd, ⁴*J*_{H-H} 2.1, ³*J*_{H-H} 8.5 Hz, 1H, H aromatic), 7.38 (d, ³*J*_{H-H} 15.5 Hz, 1H, CH=CH), 7.01 (d, ³*J*_{H-H} 8.6 Hz, 1H, H aromatic), 6.96 (d,³*J*_{H-H} 8.5 Hz, 1H, H aromatic), 3.99 (s, 3H, OCH₃), 3.96 (s, 3H, OCH₃); ¹³C NMR (75 MHz, CDCl₃, 25 °C): δ = 56.3 (O**C**H₃), 56.4 (O**C**H₃), 111.4 (**C**H aromatic), 112.0 (**C**H aromatic), 120.0 (CH**C**H), 123.0 (**C** aromatic), 123.3 (**C** aromatic), 128.4 (**C** aromatic), 128.9 (**C**H aromatic), 129.2 (**C**H aromatic), 129.5 (**C**H aromatic), 130.8 (**C**H aromatic), 131.6 (**C** aromatic), 143.1 (**C**HCH), 156.8 (**C**OMe), 158.7 (**C**OMe), 187.4 (**C**=O).

### (E)-1-(3-bromo-4-methoxyphenyl)-3-(3-chloro-4-methoxyphenyl)prop-2-en-1-one - Example 8

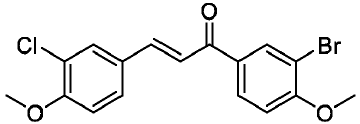

In an embodiment, 3-bromo-4-methoxyacetophenone, in particular 50 mg, 0.22 mmol, 1 equiv., and 3-chloro-4-methoxybenzaldehyde, in particular 84 mg, 0.33 mmol, 1.5 equiv., were dissolved in MeOH, in particular 5 mL of MeOH. NaOH, in particular 34 mg, 0.87 mmol, 4 equiv., was added slowly. The reaction mixture was stirred under reflux during 4 h, cooled to room temperature, poured into ice and acidified with dilute HCl. The mixture was extracted with dichloromethane, washed with water, dried over sodium sulfate, and evaporated. The residue was purified by chromatography, in particular with dichloromethane/hexane: 1/1 by TLC to give the product as a yellow solid, in particular 74 mg, with a yield of 92 %.

In an embodiment, the characterization of (*E*)-1-(3-bromo-4-methoxyphenyl)-3-(3-chloro-4-methoxyphenyl)prop-2-en-1-one is as follows: yellow solid, m.p. of 166-168 °C, ESI-MS: m/z: 383.0 [M ⁷⁹Br ³⁵Cl +H]⁺ (100 %), 381.0 [M ⁷⁹Br ³⁷Cl +H]⁺ or [M ⁸¹Br ³⁵Cl +H] (70 %), 385.0 [M ⁸¹Br³⁷Cl +H]⁺ (15 %). Anal Calcd (%) for C₁₇H₁₄BrClO₃.CH₃OH (413.69): C 52.26, H 4.39; found C 52.20, H 4.06; ¹H NMR (300 MHz, CDCl₃, 25 °C): δ = 8.26 (d, ⁴*J*_{H-H} 1.9 Hz, 1H, H aromatic), 8.02 (dd, ⁴*J*_{H-H} 1.9, ³*J*_{H-H} 7.4 Hz, 1H, H aromatic), 7.73 (d, ⁴*J*_{H-H} 2.1 Hz, 1H, H aromatic), 7.73 (d, ³*J*_{H-H} 15.6 Hz, 1H, CH=CH), 7.50 (dd, ⁴*J*_{H-H} 2.1, ³*J*_{H-H} 7.2 Hz, 1H, H aromatic), 7.38 (d, ³*J*_{H-H} 15.6 Hz, 1H, CH=CH), 6.99 (d, ³*J*_{H-H} 7.4 Hz, 1H, H aromatic), 6.96 (d,³*J*_{H-H} 7.2 Hz, 1H, H arom), 3.99 (s, 3H, OCH₃), 3.96 (s, 3H, OCH₃); ¹³C NMR (75 MHz, CDCl₃, 25 °C): δ = 56.3 (O**C**H₃), 56.5 (O**C**H₃), 111.2 (**C**H arom), 112.0 (**C**H aromatic), 112.0 (**C** aromatic), 120.0 (CH**C**H), 123.3 (**C** aromatic), 128.4 (**C** aromatic), 129.2 (**C**H aromatic), 129.5 (**C**H aromatic), 129.7 (**C**H arom), 132.1 (**C** aromatic), 134.0 (**C**H aromatic), 143.1 (**C**HCH), 156.8 (**C**OMe), 159.5 (**C**OMe), 187.2 (**C**=O).

### (E)-3-(3-bromo-4-methoxyphenyl)-1-(3-chloro-4-methoxyphenyl)prop-2-en-1-one - Example 9

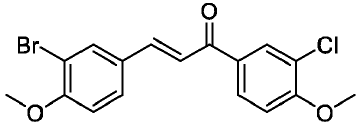

In an embodiment, 3-chloro-4-methoxyacetophenone, in particular 50 mg, 0.27 mmol, 1 equiv., and 3-bromo-4-methoxybenzaldehyde, in particular 123 mg, 0.54 mmol, 2 equiv., were dissolved in MeOH, in particular 10 mL of MeOH. NaOH (43 mg, 1.08 mmol, 4 equiv.) was added slowly. The reaction mixture was stirred under reflux during 4 h, cooled to room temperature, poured into ice and acidified with dilute HCl. The mixture was extracted with dichloromethane, washed with water, dried over sodium sulfate, and evaporated. The residue was purified by chromatography, in particular with dichloromethane/hexane: 2/1 by TLC to give the product as a yellow solid, in particular 71 mg of (*E*)-3-(3-bromo-4-methoxyphenyl)-1-(3-chloro-4-methoxyphenyl)prop-2-en-1-one, a yield of 71%.

In an embodiment, the characterization of (*E*)-3-(3-bromo-4-methoxyphenyl)-1-(3-chloro-4-methoxyphenyl)prop-2-en-1-one is as follows: yellow solid, m.p. of 148-150 °C, ESI-MS: m/z: 383.0 [M ⁷⁹Br ³⁵Cl +H]⁺ (100%), 381.0 [M ⁷⁹Br ³⁷Cl +H]⁺ or [M ⁸¹Br ³⁵Cl +H] (70%), 385.0 [M ⁸¹Br³⁷Cl +H]⁺ (15%). ¹H NMR (300 MHz, CDCl₃, 25 °C): δ = 8.09 (d, ⁴*J*_{H-H} 2.1Hz, 1H, H aromatic), 7.97 (dd, ⁴*J*_{H-H} 2.1, ³*J*_{H-H} 8.6 Hz, 1H, H aromatic), 7.90 (d, ⁴*J*_{H-H} 2.1 Hz, 1H, H aromatic), 7.72 (d, ³*J*_{H-H} 15.6 Hz, 1H, CH=CH), 7.55 (dd, ⁴*J*_{H-H} 2.1, ³*J*_{H-H} 8.5 Hz, 1H, H aromatic), 7.38 (d, ³*J*_{H-H} 15.6 Hz, 1H, CH=CH), 7.02 (d, ³*J*_{H-H} 8.6 Hz, 1H, H aromatic), 6.94 (d,³*J*_{H-H} 8.5 Hz, 1H, H aromatic), 4.00 (s, 3H, OCH₃), 3.96 (s, 3H, OCH₃). ¹³C NMR (75 MHz, CDCl₃, 25 °C): δ = 56.4 (O**C**H₃), 56.4 (O**C**H₃), 111.4 (**C**H aromatic), 111.9 (**C**H aromatic), 112.4 (**C** aromatic), 120.0 (CH**C**H), 123.0 (**C** aromatic), 128.9 (**C**H aromatic), 128.9 (**C** aromatic), 129.9 (**C**H aromatic), 130.8 (**C**H aromatic), 131.6 (**C** aromatic), 132.6 (**C**H aromatic), 143.0 (**C**HCH), 157.7 (**C**OMe), 158.7 (**C**OMe), 187.3 (**C**=O).

### (E)-1,3-bis(3-bromo-4-methoxyphenyl)prop-2-en-1-one - Example 10

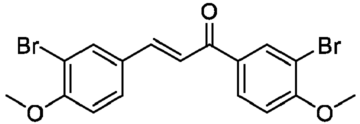

In an embodiment, 3-bromo-4-methoxyacetophenone, in particular 50 mg, 0.22 mmol, 1 equiv., and 3-bromo-4-methoxybenzaldehyde, in particular 70 mg, 0.33 mmol, 1.5 equiv., were dissolved in MeOH, in particular in 10 mL of MeOH. NaOH, in particular 35 mg, 0.87 mmol, 4 equiv. was added slowly. The reaction mixture was stirred under reflux during 4 h, cooled to room temperature, poured into ice and acidified with dilute HCl. The mixture was extracted with dichloromethane, washed with water, dried over sodium sulfate, and evaporated. The residue was purified by chromatography, in particular with dichloromethane/hexane: 2/1 by TLC to give the product as a yellow solid, in particular 68 mg, with a yield of 73%.

In an embodiment, the characterization of (*E*)-1,3-bis(3-bromo-4-methoxyphenyl)prop-2-en-1-one is as follows: yellow solid, m.p. of 152-153°C, ESI-MS: m/z: 427.0 [M ⁷⁹Br ⁷⁹Br +H]⁺ (100 %), 424.9 [M ⁷⁹Br ⁸¹Br +H]⁺ (50 %), 428.0 [M ⁸¹Br ⁸¹Br+H]⁺ (40 %). Anal Calcd (%) for C₁₇H₁₄Br₂O₃.CH₃OH (458.14): C 47.19, H 3.96; found C 47.15, H 3.57; ¹H NMR (300 MHz, CDCl₃, 25 °C): δ = 8.25 (d, ⁴*J*_{H-H} 2.1 Hz, 1H, H aromatic), 8.00 (dd, ⁴*J*_{H-H} 2.1, ³*J*_{H-H} 8.6 Hz, 1H, H aromatic), 7.89 (d, ⁴*J*_{H-H} 2.1 Hz, 1H, H aromatic), 7.71 (d, ³*J*_{H-H} 15.5 Hz, 1H, CH=CH), 7.54 (dd, ⁴*J*_{H-H} 2.1, ³*J*_{H-H} 8.5 Hz, 1H, H aromatic), 7.36 (d, ³*J*_{H-H} 15.5 Hz, 1H, CH=CH), 6.97 (d, ³*J*_{H-H} 8.6 Hz, 1H, H aromatic), 6.92 (d,³*J*_{H-H} 8.4 Hz, 1H, H aromatic), 3.98 (s, 3H, OCH₃), 3.95 (s, 3H, OCH₃); ¹³C NMR (75 MHz, CDCl₃, 25 °C): δ = 56.4 (O**C**H₃), 56.5 (O**C**H₃), 111.2 (**C**H aromatic), 111.7 (**C**H aromatic), 112.0 (**C** aromatic), 112.4 (**C** aromatic), 120.0 (CH**C**H), 128.9 (**C** aromatic), 129.7 (**C**H aromatic), 129.9 (**C**H aromatic), 132.1 (**C** aromatic), 132.7 (**C**H aromatic), 134.0 (**C**H aromatic), 143.0 (CH**C**H), 157.7 (**C**OMe), 159.5 (**C**OMe), 187.2 (**C**=O).

### (E)-3-(3-chloro-4-(dimethylamino)phenyl)-1-(3-chloro-4-methoxyphenyl)prop-2-en-1-one - Example 11

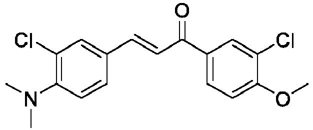

In an embodiment, 3-chloro-4-methoxyacetophenone, in particular 50 mg, 0.27 mmol, 1 equiv., and 3-chloro-4-(dimethylamino)benzaldehyde, in particular 74 mg, 0.41 mmol, 1.5 equiv. were dissolved in MeOH, in particular 10 mL of MeOH. NaOH, in particular 43 mg, 1.08 mmol, 4 equiv. was added slowly. The reaction mixture was stirred under reflux during 4 h, cooled to room temperature, poured into ice and acidified with dilute HCl. The mixture was extracted with dichloromethane, washed with water, dried over sodium sulphate, and evaporated. The residue was purified by chromatography, in particular with dichloromethane/hexane: 2/1 by TLC to give the product as a yellow solid, in particular 62 mg, yield of 66%.

In an embodiment, the characterization of (*E*)-3-(3-chloro-4-(dimethylamino)phenyl)-1-(3-chloro-4-methoxyphenyl)prop-2-en-1-one is as follows: yellow solid, m.p. 111-113 °C, ESI-MS: m/z: 350.1[M ³⁵Cl ³⁵Cl +H]⁺ (100%), 352.1 [M ³⁵Cl ³⁷Cl +H]⁺ (60%), 354.1 [M ³⁷Cl³⁷Cl +H]⁺ (10%). Anal Calcd (%) for C₁₈H₁₇Cl₂NO₂ (350.24): C 61.73, H 4.89, N 4.00; found C 61.86, H 4.90, N 3.93. ¹H NMR (300 MHz, CDCl₃, 25 °C): δ = 8.09 (d, ⁴*J*_{H-H} 2.1 Hz, 1H, H aromatic), 7.97 (dd, ⁴*J*_{H-H} 2.1, ³*J*_{H-H} 8.6 Hz, 1H, H aromatic), 7.72 (d, ³*J*_{H-H} 15.5 Hz, 1H, CH=CH), 7.67 (d, ⁴*J*_{H-H} 2.0 Hz, 1H, H aromatic), 7.46 (dd, ⁴*J*_{H-H} 2.0, ³*J*_{H-H} 8.4 Hz, 1H, H aromatic), 7.37 (d, ³*J*_{H-H} 15.5 Hz, 1H, CH=CH), 7.04 (d, ³*J*_{H-H} 8.4 Hz, 1H, H arom), 7.02 (d,³*J*_{H-H} 8.6 Hz, 1H, H aromatic), 4.00 (s, 3H, OCH₃), 2.90 (s, 6H, NCH₃); ¹³C NMR (75 MHz, CDCl₃, 25 °C): δ = 44.9 (2 × N**C**H₃), 56.5 (O**C**H₃), 111.4 (**C**H aromatic), 123.1 (CH**C**H), 125.9 (**C** aromatic), 127.6 (**C**H aromatic), 128.3 (**C**H aromatic), 128.9 (**C** aromatic), 129.0 (**C**H aromatic), 130.9 (**C**H aromatic), 131.2 (**C** aromatic), 131.3 (**C** aromatic), 131.9 (**C**H aromatic), 141.9 (**C**HCH), 151.1 (**C**NCH₃), 158.9 (**C**OCH₃), 187.0 (**C**=O).

### (E)-1-(3-bromo-4-methoxyphenyl)-3-(3-chloro-4-(dimethylamino)phenyl)prop-2-en-1-one - Example 12

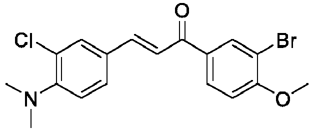

In an embodiment, 3-bromo-4-methoxyacetophenone, in particular 50 mg, 0.22 mmol, 1 equiv. and 3-chloro-4-(dimethylamino)benzaldehyde, in particular 60 mg, 0.33 mmol, 1.5 equiv. were dissolved in MeOH, in particular in 10 mL of MeOH. NaOH, in particular 35 mg, 0.87 mmol, 4 equiv., was added slowly. The reaction mixture was stirred under reflux during 4 h, cooled to room temperature, poured into ice and acidified with dilute HCl. The mixture was extracted with dichloromethane, washed with water, dried over sodium sulfate, and evaporated. The residue was purified by chromatography, in particular with dichloromethane/hexane: 2/1 by TLC to give the product as a yellow solid, in particular 62 mg, yield of 72%.

In an embodiment, the characterization of (*E*)-1-(3-bromo-4-methoxyphenyl)-3-(3-chloro-4-(dimethylamino)phenyl)prop-2-en-1-one is as follows: yellow solid, m.p. 108-110°C, ESI-MS: m/z: 396.0 [M ⁷⁹Br ³⁵Cl +H]⁺ (100 %), 394.0 [M ⁷⁹Br ³⁷Cl +H]⁺ or [M ⁸¹Br ³⁵Cl +H] (70 %), 398.0 [M ⁸¹Br³⁷Cl +H]⁺ (15 %). ¹H NMR (300 MHz, CDCl₃, 25 °C): δ = 8.25 (d, ⁴*J*_{H-H} 2.1 Hz, 1H, H aromatic), 8.00 (dd, ⁴*J*_{H-H} 2.1, ³*J*_{H-H} 8.6Hz, 1H, H aromatic), 7.71 (d, ³*J*_{H-H} 15.5 Hz, 1H, CH=CH), 7.66 (d, ⁴*J*_{H-H} 2.0 Hz, 1H, H aromatic), 7.46 (dd, ⁴*J*_{H-H} 2.0, ³*J*_{H-H} 8.4 Hz, 1H, H aromatic), 7.37 (d, ³*J*_{H-H} 15.5 Hz, 1H, CH=CH), 7.03 (d, ³*J*_{H-H} 8.4 Hz, 1H, H aromatic), 6.97 (d,³*J*_{H-H} 8.6 Hz, 1H, H aromatic), 3.98 (s, 3H, OCH₃), 2.90 (s, 6H, NCH₃); ¹³C NMR (75 MHz, CDCl₃, 25 °C): δ = 44.0 (NCH₃), 44.1 (NCH₃), 56.6 (O**C**H₃), 111.3 (CH aromatic), 112.1 (C aromatic), 112.1 (C aromatic), 117.4 (C aromatic), 121.3 (CHCH), 128.9 (CH aromatic), 128.9(CH aromatic), 129.7(CH aromatic), 131.9 (CH aromatic), 132.0 (C aromatic), 134.0 (CH aromatic), 142.3 (CHCH), 150.5 (CNCH₃), 159.6 (COCH₃), 187.0 (C=O).

### (E)-3-(3-bromo-4-(dimethylamino)phenyl)-1-(3-chloro-4-methoxyphenyl)prop-2-en-1-one - Example 13

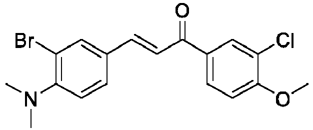

In an embodiment, 3-chloro-4-methoxyacetophenone, in particular 50 mg, 0.27 mmol, 1 equiv., and 3-bromo-4-(dimethylamino)benzaldehyde, in particular 123 mg, 0.54 mmol, 2 equiv. were dissolved in MeOH, in particular were dissolved in 10 mL of MeOH. NaOH, in particular 43 mg, 1.08 mmol, 4 equiv. was added slowly. The reaction mixture was stirred under reflux during 4 h, cooled to room temperature, poured into ice and acidified with dilute HCl. The mixture was extracted with dichloromethane, washed with water, dried over sodium sulphate, and evaporated. The residue was purified by chromatography, in particular with dichloromethane/hexane: 2/1 on TLC to give the product as a yellow solid, in particular 63 mg, yield of 63%.

In an embodiment, the characterization of (*E*)-3-(3-bromo-4-(dimethylamino)phenyl)-1-(3-chloro-4-methoxyphenyl)prop-2-en-1-one is as follows: yellow solid, m.p. of 125-127 °C, ESI-MS: m/z: 396.0 [M ⁷⁹Br ³⁵Cl + H]⁺ (100%), 394.0 [M ⁷⁹Br ³⁷Cl +H]⁺ or [M ⁸¹Br ³⁵Cl +H] (70%), 398.0 [M ⁸¹Br³⁷Cl +H]⁺ (15%). Anal Calcd (%) for C₁₈H₁₇BrClNO₂ (394.69): C 54.78, H 4.34, N 3.55; found C 54.58, H 4.48, N 3.52. ¹H NMR (300 MHz, CDCl₃, 25 °C): δ = 8.09 (d, ⁴*J*_{H-H} 2.3 Hz, 1H, H aromatic), 7.97 (dd, ⁴*J*_{H-H} 2.3, ³*J*_{H-H} 8.6 Hz, 1H, H aromatic), 7.88 (d, ⁴*J*_{H-H} 2.1 Hz, 1H, H aromatic), 7.72 (d, ³*J*_{H-H} 15.5 Hz, 1H, CH=CH), 7.51 (dd, ⁴*J*_{H-H} 2.1, ³*J*_{H-H} 8.2 Hz, 1H, H aromatic), 7.38 (³*J*_{H-H} 15.5 Hz, 1H, CH=CH), 7.06 (d, ³*J*_{H-H} 8.2 Hz, 1H, H atom), 7.02 (d,³*J*_{H-H} 8.6 Hz, 1H, H aromatic), 4.00 (s, 3H, OCH₃), 2.89 (s, 6H, NCH₃); ¹³C NMR (75 MHz, CDCl₃, 25 °C): δ = 44.1 (N**C**H₃), 56.4 (O**C**H₃), 111.4 (**C**H aromatic), 120.0 (CH**C**H), 123.1 (**C** aromatic), 128.8 (**C**H aromatic), 128.8 **(C** aromatic), 129.0 (**C**H aromatic), 130.8(**C**H aromatic), 130.8 (**C**H aromatic), 131.3 **(C** aromatic), 131.4 (CH aromatic), 134.4 **(C** aromatic), 142.0 (**C**HCH), 152.8 (CNCH₃), 158.8 (**C**OCH₃), 187.1 (**C**=O).

### (E)-3-(3-bromo-4-(dimethylamino)phenyl)-1-(3-bromo-4-methoxyphenyl)prop-2-en-1-one - Example 14

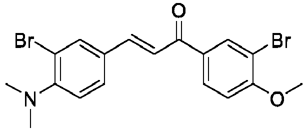

In an embodiment, 3-bromo-4-methoxyacetophenone, in particular 50 mg, 0.22 mmol, 1 equiv., and 3-bromo-4-(dimethylamino)benzaldehyde, in particular 75 mg, 0.33 mmol, 1.5 equiv., were dissolved in MeOH, in particular in 10 mL of MeOH. NaOH, in particular 35 mg, 0.87 mmol, 4 equiv. was added slowly. The reaction mixture was stirred under reflux during 4 h, cooled to room temperature, poured into ice and acidified with dilute HCl. The mixture was extracted with dichloromethane, washed with water, dried over sodium sulfate, and evaporated. The residue was purified by chromatography, in particular with dichloromethane/hexane: 2/1 by TLC to give the product as a yellow solid, in particular 67 mg of the (*E*)-3-(3-bromo-4-(dimethylamino)phenyl)-1-(3-bromo-4-methoxyphenyl)prop-2-en-1-one, yield of 70%.

In an embodiment, the characterization of (*E*)-3-(3-bromo-4-(dimethylamino)phenyl)-1-(3-bromo-4-methoxyphenyl)prop-2-en-1-one is as follows: yellow solid, m.p. of 124-125°C, ESI-MS: m/z: 440.0 [M ⁷⁹Br ⁷⁹Br +H]⁺ (100 %), 438.0 [M ⁷⁹Br ⁸¹Br +H]⁺ (45 %), 442.0 [M ⁸¹Br ⁸¹Br+H]⁺ (40 %). Anal Calcd (%) for C₁₈H₁₇Br₂NO₂.CH₃OH (471.18): C 48.43, H 4.49, N 2.97; found C 48.31, H 4.06, N 3.11. ¹H NMR (300 MHz, CDCl₃, 25 °C): δ = 8.26 (d, ⁴*J*_{H-H} 2.1 Hz, 1H, H aromatic), 8.01 (dd, ⁴*J*_{H-H} 2.1, ³*J*_{H-H} 8.6 Hz, 1H, H aromatic), 7.87 (d, ⁴*J*_{H-H} 2.0 Hz, 1H, H aromatic), 7.71 (d, ³*J*_{H-H} 15.5 Hz, 1H, CH=CH), 7.51 (dd, ⁴*J*_{H-H} 2.0, ³*J*_{H-H} 8.4 Hz, 1H, H aromatic), 7.37 (d, ³*J*_{H-H} 15.5 Hz, 1H, CH=CH), 7.05 (d, ³*J*_{H-H} 8.4 Hz, 1H, H arom), 6.98 (d,³*J*_{H-H} 8.6 Hz, 1H, H arom), 3.99 (s, 3H, OCH₃), 2.89 (s, 6H, NCH₃); ¹³C NMR (75 MHz, CDCl₃, 25 °C): δ = 44.0 (N**C**H₃), 44.1 (N**C**H₃), 56.6 (O**C**H₃), 111.3 (**C**H aromatic), 112.0 **(C** arom), 112.1 **(C** aromatic), 117.4 **(C** aromatic), 121.3 (CH**C**H), 128.9 (**C**H aromatic), 128.9(**C**H arom), 129.7(**C**H aromatic), 131.9 (**C**H aromatic), ,134.0 **(C** aromatic), 134.2 (**C**H aromatic), 142.3 (**C**HCH), 146.4 (**C**NCH₃), 159.6 (**C**OCH₃), 187.0 (**C**=O).

The synthesis and characterization of 3-chloro-4-methoxybenzaldehyde - example 15 and 3-bromo-4-methoxybenzaldehyde - example 16 are now disclosed.

### 3-chloro-4-methoxybenzaldehyde - Example 15

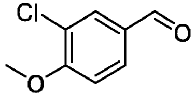

In an embodiment, a mixture of *p*-methoxybenzaldehyde, in particular 1.00 mL, 8.2 mmol, 1 equiv., and ammonium nitrate, in particular 0.13 g, 1.6 mmol, 0.2 equiv. in acetonitrile, in particular 10 mL of acetonitrile was stirred at room temperature. N-Chlorosuccinimide, in particular 4.36 g, 33 mmol, 4 equiv. was added slowly over 3 days. The solid obtained was filtered and the mixture was extracted with dichloromethane, washed with water and dried over Na₂SO₄. The solvent was then evaporated under reduced pressure, and silica gel flash column chromatography, in particular using as an eluent a mixture of dichloromethane/hexane: 1/1, of the residue gave the product as a white solid, in particular 0.62 g of 3-chloro-4-methoxybenzaldehyde, yield of 45%.

In an embodiment, the characterization of 3-chloro-4-methoxybenzaldehyde is as follows: white solid, m.p of 60-62 °C, ¹H NMR (300 MHz, CDCl₃, 25 °C): δ =9.84 (s, CHO) 7.84 (d, ⁴*J*_{H-H} 2.0 Hz, 1H, H aromatic), 7.77 (dd, ⁴*J*_{H-H} 2.0, ³*J*_{H-H} 8.5 Hz, 1H, H aromatic), 7.04 (d, ³*J*_{H-H} 8.5 Hz, 1H, H aromatic), 3.99 (s, 3H, OCH₃).

### 3-bromo-4-methoxybenzaldehyde - Example 16

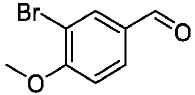

In an embodiment, a mixture of *p*-methoxybenzaldehyde, in particular 1.25 mL, 10 mmol, 1 equiv. and ammonium nitrate, in particular 0.16 g, 2 mmol, 0.2 equiv. in acetonitrile, in particular in 10 mL of acetonitrile was stirred at room temperature. N-Bromosuccinimide, in particular 3.56 g, 20 mmol, 2 equiv. was added slowly over 3 days. The solid obtained was filtered and the mixture was extracted, in particular with dichloromethane, washed with water, dried, and evaporated. The residue was purified by chromatography, in particular using as an eluent dichloromethane, on TLC to give the product as orange solid, in particular 0.92 g of 3-bromo-4-methoxybenzaldehyde, yield of 43%.

In an embodiment, the characterization of 3-bromo-4-methoxybenzaldehyde is as follows: orange solid, m.p of 55-57 °C, ¹H NMR (300 MHz, CDCl₃, 25 °C): ^{™} = 9.85 (s, 1H, CHO), 8.09 (d, ⁴*J*_{H-H} 1.9 Hz, 1 H, H aromatic), 7.83 (dd, ⁴*J*_{H-H} 1.9, ³*J*_{H-H} 8.7 Hz, 1H, H aromatic), 7.02 (d, ³*J*_{H-H} 8.7 Hz, 1H, H aromatic), 4.00 (s, 3 H, OC*H*₃).

The synthesis and characterization of 3-chloro-4-(dimethylamino)benzaldehyde - example 17 and 3-bromo-4-(dimethylamino)benzaldehyde - example 18 are now disclosed.

### 3-chloro-4-(dimethylamino)benzaldehyde - Example 17

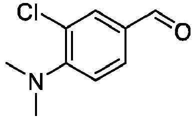

In an embodiment, *N*-Chlorosuccinimide in particular 2.67 g, 20 mmol, 2 equiv. was added to a solution of 4-(dimethylamino)benzaldehyde, in particular 1.49 g, 10 mmol, 1 equiv. in dichloromethane, in particular 50 mL of dichloromethane, and the reaction mixture was stirred at room temperature for 72 h. The solvent was then evaporated under reduced pressure, and silica gel flash column chromatography, in particular using as eluent dichloromethane/petroleum ether: 1/1, of the residue gave the product as a colorless liquid, in particular 0.98 g of 3-chloro-4-(dimethylamino)benzaldehyde was obtained with a yield of 54%.

In an embodiment, the characterization of 3-chloro-4-(dimethylamino)benzaldehyde is as follows: colorless liquid, ¹H NMR (300 MHz, CDCl₃, 25 °C): δ = 9.81 (s, 1H, C*H*O), 7.83 (d, ⁴*J*_{H-H} 2.1 Hz, 1 H, H aromatic), 7.68 (dd, ⁴*J*_{H-H} 2.1, ³*J*_{H-H} 8.4 Hz, 1H, H aromatic), 7.05 (d, ³*J*_{H-H} 8.4 Hz, 1H, H aromatic), 2.96 (s, 6 H, C*H*₃).

### 3-bromo-4-(dimethylamino)benzaldehyde - Example 18

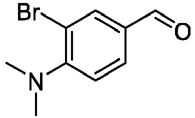

In an embodiment, N-Bromosuccinimide, in particular 1.78 g, 10 mmol, 1 equiv. was added to a solution of 4-(dimethylamino)benzaldehyde, in particular 1.49 g, 10 mmol, 1 equiv. in dichloromethane, in particular 50 mL of dichloromethane, and the reaction mixture was stirred at room temperature for 72 h. The solvent was then evaporated under reduced pressure, and silica gel flash column chromatography, in particular using as eluent a mixture of dichloromethane/petroleum ether: 1/1, of the residue gave the product as a colorless liquid, in particular 1.62 g of 3-bromo-4-(dimethylamino)benzaldehyde, yield of 71%.

In an embodiment, the characterization of 3-bromo-4-(dimethylamino)benzaldehyde is as follows: colourless liquid, ¹H NMR (300 MHz, CDCl₃, 25 °C): δ = 9.80 (s, 1H, C*H*O), 8.03 (d, ⁴*J*_{H-H} 1.8 Hz, 1 H, H aromatic), 7.73 (dd, ⁴*J*_{H-H} 1.8, ³*J*_{H-H} 8.4 Hz, 1 H, H aromatic), 7.06 (d, ³*J*_{H-H} 8.4 Hz, 1H, H aromatic), 2.94 (s, 6 H, C*H*₃).

The synthesis and characterization of 1-(3-chloro-4-methoxyphenyl)ethan-1-one - example 19 and 1-(3-bromo-4-methoxyphenyl)ethan-1-one - example 20 are now disclosed.

### 1-(3-chloro-4-methoxyphenyl)ethan-1-one - Example 19

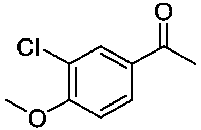

In an embodiment, to a solution of 4'-methoxyacetophenone, in particular 1.0 g, 6.66 mmol in glacial acetic acid, in particular 8 mL of glacial acetic acid was added to 5% NaOCl in water, in particular to 30 mL of 5% NaOCl in water, dropwise. The mixture was stirred for 30 minutes. A solid was obtained, which was separated by filtration and purified over silica gel flash column chromatography, in particular using a mixture of dichloromethane/petroleum ether 3/1. The solvent was completely evaporated, yielding 1-(3-chloro-4-methoxyphenyl)ethan-1-one as white crystals, in particular 0.568 g and 3.08 mmol of 1-(3-chloro-4-methoxyphenyl)ethan-1-one, yield of 46%.

In an embodiment, the characterization of 1-(3-chloro-4-methoxyphenyl)ethan-1-one is as follows: white crystals, m.p. of 80-82 °C, ¹H NMR (300 MHz, CDCl₃, 25 °C): δ = 8.00 (d, ⁴*J*_{H-H} 2.2 Hz, 1H, H aromatic), 7.87 (dd, ⁴*J*_{H-H} 2.2, ³*J*_{H-H} 8.4 Hz, 1H, H aromatic), 6.97 (d, ³*J*_{H-H} 8.4 Hz, 1H, H aromatic), 3.97 (s, 3H, OCH₃), 2.56 (s, 3H, CCH₃).

### 1-(3-bromo-4-methoxyphenyl)ethan-1-one - Example 20

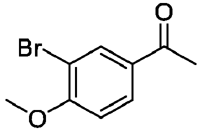

In an embodiment, a mixture of *p*-methoxyacetophenone, in particular 1.00 g, 6.6 mmol, 1 equiv. of *p*-methoxyacetophenone and ammonium nitrate, in particular 0.10 g, 1.3 mmol, 0.2 equiv. of ammonium nitrate in acetonitrile, in particular in 10 mL of acetonitrile was stirred at room temperature. *N*-Bromosuccinimide, in particular 3.56 g, 20 mmol, 2 equiv. was added slowly over 3 days. The solid obtained was filtered and the mixture was extracted with dichloromethane, washed with water and dried over Na₂SO₄. The solvent was then evaporated under reduced pressure, and silica gel flash column chromatography, in particular an eluent mixture of dichloromethane/ hexane: 1/1 of the residue gave the product as a white solid, in particular 1.07 g of 1-(3-bromo-4-methoxyphenyl)ethan-1-one and a yield of 71%).

In an embodiment, the characterization of 1-(3-bromo-4-methoxyphenyl)ethan-1-one is as follows: white solid, m.p. of 90-92 °C, ¹H NMR (300 MHz, CDCl₃, 25 °C): δ = 8.17 (d, ⁴*J*_{H-H} 2.1 Hz, 1H, H aromatic), 7.93 (dd, ⁴*J*_{H-H} 2.1, ³*J*_{H-H} 8.6 Hz, 1H, H aromatic), 6.95 (d, ³*J*_{H-H} 8.6 Hz, 1H, H aromatic), 3.98 (s, 3H, OCH₃), 2.57 (s, 3H, CCH₃).

The synthesis and characterization of (*E*)-4-[3-bromo-4-(dimethylamino)phenil]but-3-en-2-one - example 21, (*E*)-4-[3-cloro-4-(dimethylamino)phenil]but-3-en-2-one - example 22 are now disclosed.

### (E)-4-[3-bromo-4-(dimethylamino)phenil]but-3-en-2-one - Example 21

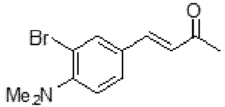

In an embodiment, to a solution of 3-bromo-4-(dimethylamino)benzaldehyde, in particular 219 mg, 0.8 mmol of 3-bromo-4-(dimethylamino)benzaldehyde in methanol, in particular 10 mL of methanol was added NaOH, in particular 20 mg, 0.5 mmol of NaOH. This solution was added dropwise to an excess of acetone, in particular 10 mL, 136 mmol of acetone. After refluxing for 2-3 hours, water was added and the methanol and acetone were evaporated. The aqueous phase was extracted with dichloromethane, which upon evaporation gave 0.129 g, 0.48 mmol of (*E*)-4-[3-bromo-4-(dimethylamino)phenil]but-3-en-2-one, as an orange oil with a yield of 60%.

In an embodiment, the characterization of (*E*)-4-[3-bromo-4-(dimethylamino)phenil]but-3-en-2-one is as follows: orange oil, ¹H NMR (300 MHz, CDCl₃, 25 °C): δ = 2.36 (s, 3H, CC**H**₃ ), 2.87 (s, 6H, N(C**H**₃)₂), 6.61 (d, 1H, ³*J*_{H-H} 16.2 Hz, CHC**H**), 7.04 (d, 1H, ³*J*_{H-H} 8.1 Hz, C**H** aromatic), 7.40 (d, 1H, ³*J*_{H-H} 16.2 Hz, CH**C**H), 7.43 (dd, 1H, ³*J*_{H-H} 8.1 Hz, ⁴*J*_{H-H} 2.5 Hz, C**H** aromatic), 7.74 (d, 1H, ⁴*J*_{H-H} 2.5 Hz, C**H** aromatic), ¹³C NMR (75 MHz, CDCl₃, 25 °C): δ = 27.5 (C**C**H₃, 43.7 (N(**C**H₃)₂), 118.1 (**C**Br), 120.1 (**C**H aromatic), 125.9 (CH**C**H), 128.2 (**C**H aromatic), 129.5 **(C** aromatic), 133.8 (**C**H aromatic), 141.7 (**C**HCH), 153.6 (**C**N(CH₃)₂), 198.1 (**C**=O).

### (E)-4-[3-cloro-4-(dimethylamino)phenil]but-3-en-2-one - Example 22

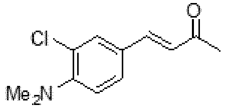

In an embodiment, to a solution of 3-chloro-4-(dimethylamino)benzaldehyde, in particular 200 mg, 1.09 mmol 3-chloro-4-(dimethylamino)benzaldehyde in methanol, in particular 10 mL 3-chloro-4-(dimethylamino)benzaldehyde was added NaOH (43.6 mg, 1.09 mmol). This solution was added dropwise to an excess of acetone, in particular 10 mL, 136 mmol of acetone. After refluxing for 2-3 hours, water was added and the methanol and acetone were evaporated. The aqueous phase was extracted, in particular with dichloromethane, which upon evaporation gave 0.142 g, 0.63 mmol of (*E*)-4-[3-cloro-4-(dimethylamino)phenil]but-3-en-2-one and a yield of 58% as a yellow oil.

In an embodiment, the characterization of (*E*)-4-[3-cloro-4-(dimethylamino)phenil]but-3-en-2-one is as follows: yellow oil, ¹H NMR (300 MHz, CDCl₃, 25 °C): δ = 2.35 (s, 3H, CC**H**₃), 2.88 (s, 6H, N(C**H**₃)₂), 6.60 (d, 1H, ³*J*_{H-H} 16.2 Hz, CHC**H**) 7.01 (d, 1H, ³*J*_{H-H} 8.4 Hz, C**H** aromatic), 7.40 (d, 1H, ³*J*_{H-H} 16.2 Hz, CH**C**H), 7.37 (dd, 1H, ³*J*_{H-H} 8.4 Hz, ⁴*J*_{H-H} 2.1 Hz, C**H** aromatic), 7.54 (d, 1H, ⁴*J*_{H-H} 2.1 Hz, C**H** aromatic), ¹³C NMR (75 MHz, CDCl₃, 25 °C): δ = 27.4 (C**C**H₃), 43.2 (N(**C**H₃)₂), 119.5 (**C**H aromatic), 125.6 (CH**C**H), 127.3 (**C**Br), 127.6 (**C**H aromatic), 128.6 **(C** aromatic), 130.4 (**C**H aromatic), 141.8 (**C**HCH), 152.0 (**C**N(CH₃)₂), 198.1 (**C**=O).

The disclosure should not be seen in any way restricted to the embodiments described and a person with ordinary skill in the art will foresee many possibilities to modifications thereof.

The above described embodiments are combinable. The following claims further set out particular embodiments of the disclosure.

## Claims

1. A compound, acceptable salt, ester, solvate thereof of formula I 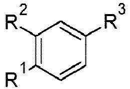 wherein
R¹ is selected from the following list: N(CH₃)₂;
R² is selected from the following list: H or halogen;
R³ is selected from the following list: 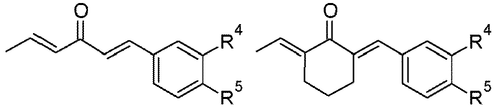
R⁴ is selected from the following list: H or halogen;
R⁵ is selected from the following list: N(CH₃)₂ or OCH₃ and
R¹, R², R³, R⁴, R⁵ are independently selected;
provided that when R¹ is N(CH₃)₂ and R² is H, R³ is different from 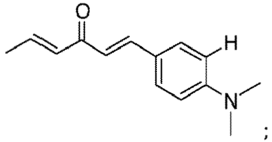
for use in the diagnostics of chronic stress, a lipid-related metabolic disease, lipid-related metabolic disorder, a demyelinating disease or demyelinating disorder;
wherein the demyelinating disease or demyelinating disorder is selected from the following list: multiple sclerosis, acute disseminated encephalomyelitis, Balo's disease, HTLV-I associated myelopathy, neuromyelitis optica, Schilder's disease, transverse myelitis, or cobalamin-deficiencies.

2. The compound according to the previous claim for use in the diagnostics of chronic stress, a lipid-related metabolic disease, lipid-related metabolic disorder.

3. The compound for use according to the previous claims, wherein the demyelinating disease or demyelinating disorder is selected from the following list: multiple sclerosis, acute disseminated encephalomyelitis, Balo's disease, HTLV-I associated myelopathy, neuromyelitis optica, Schilder's disease, transverse myelitis, or cobalamin-deficiencies.

4. The compound for use according to the previous claims, wherein the lipid-related metabolic disease or lipid-related metabolic disorder is selected from the following list: Gaucher's disease, Tay-Sachs disease, Niemann-Pick disease, Fabry's disease, fatty acid oxidation disorders, abetalipoproteinemia, apolipoprotein B deficiency, chylomicron retention disease, familial dysbetalipoproteinemia, familial hypercholesterolemia, familial hypertriglyceridemia, inborn error of lipid metabolism, hypoalphalipoproteinemia, hypobetalipoproteinemia, lipomatosis, hypertriglyceridemia, hypercholesterolemia, hypolipoproteinemia, lecithin cholesterol acyltransferase deficiency, lipoprotein lipase deficiency, Tangier disease, Zellweger spectrum disorder, adrenoleukodystrophy, Refsum disease or Urbach-Wiethe disease.

5. The compound for use according to the previous claims, wherein R¹ is N(CH₃)₂.

6. The compound for use according to the previous claims, wherein R² is H, Br or Cl.

7. The compound for use according to the previous claims, wherein R⁴ is H, Br or Cl.

8. The compound for use according to the previous claims, wherein R⁵ is N(CH₃)₂.

9. The compound for use according to the claims 2-8 wherein the compound is (1*E,*4*E*)-1,5-bis[3-chloro-4-(dimethylamino)phenyl]penta-1,4-dien-3-one; (1*E*,4*E*)-1,5-bis[3-bromo-4-(dimethylamino)phenyl]penta-1,4-dien-3-one; (2*E*,6*E*)-2,6-bis[3-chloro-4-(dimethylamino)benzylidene]cyclohexanone; or (2*E*,6*E*)-2,6-bis[3-bromo-4-(dimethylamino)benzylidene]cyclohexanone.

## Patentansprüche

1. Eine Verbindung, ein verträgliches Salz, Ester oder Solvat davon der Formel I 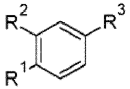 wobei
R¹ aus der folgenden Liste ausgewählt wird: N(CH₃)₂;
R² aus der folgenden Liste ausgewählt wird: H oder Halogen;
R³ aus der folgenden Liste ausgewählt wird: 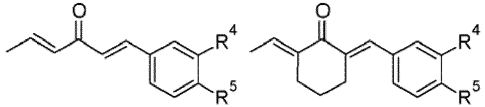
R⁴ aus der folgenden Liste ausgewählt wird: H oder Halogen;
R⁵ aus der folgenden Liste ausgewählt wird: N(CH₃)₂ oder OCH₃; und
R¹, R², R³, R⁴, R⁵ unabhängig voneinander ausgewählt werden;
vorausgesetzt, dass, wenn R¹ N(CH₃)₂ ist und R² H ist, R³ verschieden ist von 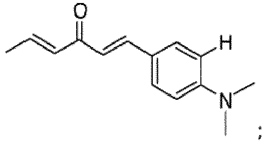
zur Verwendung bei der Diagnostik von chronischem Stress, einer lipidbezogenen Stoffwechselerkrankung, einer lipidbezogenen Stoffwechselstörung, einer demyelinisierenden Erkrankung oder einer demyelinisierenden Störung;
wobei die demyelinisierende Erkrankung oder demyelinisierende Störung aus der folgenden Liste ausgewählt wird: Multiple Sklerose, akute disseminierte Enzephalomyelitis, Morbus Baló, HTLV-I-assoziierte Myelopathie, Neuromyelitis optica, Morbus Schilder, transversale Myelitis oder Cobalamin-Mangel.

2. Die Verbindung nach dem vorangehenden Anspruch zur Verwendung in der Diagnostik von chronischem Stress, einer lipidbezogenen Stoffwechselerkrankung, einer lipidbezogenen Stoffwechselstörung.

3. Die Verbindung zur Verwendung nach dem vorangehenden Ansprüchen, wobei die demyelinisierende Erkrankung oder demyelinisierende Störung aus der folgenden Liste ausgewählt wird: Multiple Sklerose, akute disseminierte Enzephalomyelitis, Morbus Baló, HTLV-I-assoziierte Myelopathie, Neuromyelitis optica, Morbus Schilder, transversale Myelitis oder Cobalamin-Mangel.

4. Die Verbindung zur Verwendung nach den vorangehenden Ansprüchen, wobei die lipidbezogene Stoffwechselerkrankung oder lipidbezogene Stoffwechselstörung aus der folgenden Liste ausgewählt wird: Morbus Gaucher, Morbus Tay Sachs, Niemann-Pick-Krankheit, Morbus Fabry, Störungen der Fettsäure-Oxidation, Abetalipoproteinämie, Apolipoprotein-B-Mangel, Chylomikron-Retentions-Krankheit, familiäre Dysbetalipoproteinämie, familiäre Hypercholesterinämie, familiäre Hypertriglyceridämie, angeborene Fettstoffwechselstörung, Hypoalphalipoproteinämie, Hypobetalipoproteinämie, Lipomatose, Hypertriglyceridämie, Hypercholesterinämie, Hypolipoproteinämie, Lecithin-Cholesterin-Acyltransferase-Mangel, Lipoproteinlipase-Mangel, Tangier-Krankheit, Störungen des Zellweger-Spektrums, Adrenoleukodystrophie, Refsum-Krankheit oder Urbach-Wiethe-Syndrom.

5. Die Verbindung zur Verwendung nach den vorangehenden Ansprüchen, wobei R¹ N(CH₃)₂ ist.

6. Die Verbindung zur Verwendung nach den vorangehenden Ansprüchen, wobei R² H, Br oder CI ist.

7. Die Verbindung zur Verwendung nach den vorangehenden Ansprüchen, wobei R⁴ H, Br oder CI ist.

8. Die Verbindung zur Verwendung nach den vorangehenden Ansprüchen, wobei R⁵ N(CH₃)₂ ist.

9. Die Verbindung zur Verwendung nach den Ansprüchen 2-8, wobei die Verbindung (*1E,4E*)-1,5-Bis[3-chloro-4-(dimethylamino)phenyl]penta-1,4-dien-3-on; (*1E,4E*)-1,5-Bis[3-bromo-4-(dimethylamino)phenyl]penta-1,4-dien-3-on; (*2E,6E*)-2,6-Bis[3-chloro-4-(dimethylamino)benzylidene]cyclohexanon; oder (*2E,6E*)-2,6-Bis[3-bromo-4-(dimethylamino)benzylidene]cyclohexanon ist.

## Revendications

1. Un composé, sel, ester, solvate acceptable de celui-ci de formule I 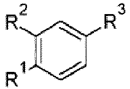 dans lequel
R¹ est sélectionné à partir de la liste suivante: N(CH₃)₂;
R² est sélectionné à partir de la liste suivante: H ou halogène;
R³ est sélectionné à partir de la liste suivante: 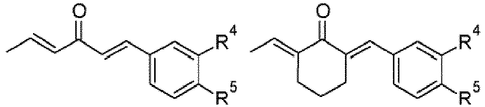
R⁴ est sélectionné à partir de la liste suivante: H ou halogène ;
R⁵ est sélectionné à partir de la liste suivante: N(CH₃)₂ ou OCH₃ et
R¹, R², R³, R⁴, R⁵ sont indépendamment sélectionnés ;
à condition que lorsque R¹ est N(CH₃)₂ et R² est H, R³ soit différent de 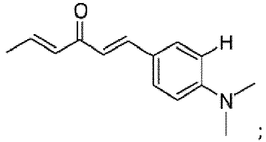
pour utiliser dans le diagnostic de stress chronique, d'une maladie métabolique liée aux lipides, d'un trouble métabolique lié aux lipides, d'une maladie démyélinisante ou d'un trouble démyélinisant;
dans lequel la maladie démyélinisante ou le trouble démyélinisant est sélectionné(e) à partir de la liste suivante: sclérose en plaques, encéphalomyélite aiguë disséminée, maladie de Balo, myélopathie associée au HTLV-I, neuromyélite optique, maladie de Schilder, myélite transverse, ou carences en cobalamine.

2. Le composé selon la revendication précédente pour utiliser dans le diagnostic de stress chronique, d'une maladie métabolique liée aux lipides, d'un trouble métabolique lié aux lipides.

3. Le composé pour utiliser selon les revendications précédentes, dans lequel la maladie démyélinisante ou le trouble démyélinisant est sélectionné(e) à partir de la liste suivante: sclérose en plaques, encéphalomyélite aiguë disséminée, maladie de Balo, myélopathie associée au HTLV-I, neuromyélite optique, maladie de Schilder, myélite transverse, ou carences en cobalamine.

4. Le composé pour utiliser selon les revendications précédentes, dans lequel la maladie métabolique liée aux lipides ou le trouble métabolique lié aux lipides est sélectionné(e) à partir de la liste suivante: maladie de Gaucher, maladie de Tay-Sachs, maladie de Niemann-Pick, maladie de Fabry, troubles d'oxydation des acides gras, abêtalipoprotéinémie, carence en apolipoprotéine B, maladie de rétention des chylomicrons, dysbêtalipoprotéinémie familiale, hypercholestérolémie familiale, hypertriglycéridémie familiale, erreur innée du métabolisme des lipides, hypoalphalipoprotéinémie, hypobêtalipoprotéinémie, lipomatose, hypertriglycéridémie, hypercholestérolémie, hypolipoprotéinémie, carence en lécithine-cholestérol acyltransférase, carence en lipoprotéine lipase, maladie de Tangier, trouble du spectre de Zellweger, adrénoleucodystrophie, maladie de Refsum ou maladie d'Urbach-Wiethe.

5. Le composé pour utiliser selon les revendications précédentes, dans lequel R¹ est N(CH₃)₂.

6. Le composé pour utiliser selon les revendications précédentes, dans lequel R² est H, Br ou CI.

7. Le composé pour utiliser selon les revendications précédentes, dans lequel R⁴ est H, Br ou CI.

8. Le composé pour utiliser selon les revendications précédentes, dans lequel R⁵ est N(CH₃)₂.

9. Le composé pour utiliser selon les revendications 2-8 dans lequel le composé est (1*E*,4*E*)-1,5-bis[3-chloro-4-(diméthylamino)phényl]penta-1,4-dièn-3-one; (1*E*,4*E*)-1,5-bis[3-bromo-4-(diméthylamino)phényl]penta-1,4-dièn-3-one; (2*E*,6*E*)-2,6-bis[3-chloro-4-(diméthylamino)benzylidène]cyclohexanone ; ou (2*E*,6*E*)-2,6-bis[3-bromo-4-(diméthylamino)benzylidène]cyclohexanone.
